# EUROPÄISCHE PATENTSCHRIFT

(11) **EP 4 366 800 B1**
(45) Veröffentlichungstag und Bekanntmachung des Hinweises auf die Patenterteilung: **26.08.2026**
(21) Anmeldenummer: 22769675.4
(22) Anmeldetag: 29.08.2022
(51) Int. Cl.: A61M 5/31, A61M 39/10, A61M 3/02

(54) **SPRITZENVORRICHTUNG**
SYRINGE DEVICE
DISPOSITIF SERINGUE

(30) Priorität: 30.08.2021 EP 21193836; 20.01.2022 EP 22152454
(43) Veröffentlichungstag der Anmeldung: 15.05.2024
(73) Patentinhaber: MEDICE Pharma GmbH, 5400 Hallein (AT)
(72) Erfinder: AMMER, Richard, 58644 Iserlohn (DE)
(74) Vertreter: Isarpatent
(86) Internationale Anmeldenummer: PCT/EP2022/073917
(87) Internationale Veröffentlichungsnummer: WO 2023/031100

(56) Entgegenhaltungen:
- EP-A1- 3 659 594
- ES-U- 27 122
- US-A- 6 165 168
- US-A1- 2003 195 478
- US-A1- 2017 014 535

## Beschreibung

### Gebiet der Erfindung

Die vorliegende Erfindung betrifft eine Spritzenvorrichtung, mit vorzugsweise zumindest einem herkömmlichen Standardvolumen (üblicherweise im Bereich von 1ml, 2ml, 5ml, 10ml bis 20ml) und einem integrierten, trichterförmigen Katheter-Verbindungsadapter, insbesondere zum Einsatz bei urologischen Katheterisierungen.

### Hintergrund der Erfindung

Die Entleerung der Blase wird durch das autonome Nervensystem und die Miktionszentren im Gehirn gesteuert.

Jede Störung der Nervenbahnen, z. B. verursacht durch eine Rückenmarksverletzung, eine Entzündung, Multiple Sklerose, eine Reizblase usw., kann zu Schwierigkeiten bei der Blasenentleerung führen. Bei der intermittierenden Katheterisierung wird mehrmals am Tag ein Schlauch eingeführt, um die Blase zu entleeren. Die erneute Katheterisierung erfolgt in regelmäßigen Abständen, um die Blasenfunktion des Füllens und Entleerens zu imitieren. Der intermittierende Katheterismus ist ein bevorzugtes Blasenmanagementverfahren für Personen mit Harnverhalt, Blasenobstruktion, einem katheterisierbaren Kanal nach einer Harnableitung, neurogenen Funktionsstörungen des unteren Harntrakts aufgrund von Erkrankungen, die einen Gefühlsverlust verursachen, der eine assistierte Blasenentleerung erfordert.

Es können einzelne sterile Einwegkatheter und Katheter-Sets verwendet werden, die in der Regel über eigene Auffangbeutel verfügen. Neuere Kathetermaterialien, die für eine einfache und bequeme Handhabung entwickelt wurden, haben dazu beigetragen, die Lebensqualität derjenigen zu verbessern, die für das Blasenmanagement auf den intermittierenden Katheterismus angewiesen sind.

Bei der Auswahl des Katheters spielt die Benutzerfreundlichkeit für den Patienten eine wichtige Rolle. Weitere wichtige Faktoren sind der Lebensstil des Patienten, die Funktion der Hände und die Fähigkeiten des Pflegepersonals. Zur Vorbeugung von Katheter-assoziierten Harnwegsinfektionen ist das Verständnis von zahlreichen Faktoren erforderlich im Zusammenhang mit der Neuroanatomie, der Blasenfunktion, den Behandlungsmethoden und den medizinischen Möglichkeiten.

Bei der intermittierenden Katheterisierung (kurz IK) wir ein als "Katheter" bezeichneter Schlauch eingesetzt, mit dem der Urin von der Blase nach außen geleitet wird. Dabei führt der Patient diesen Katheter in die Harnröhre ein und von dort in die Blase, um Urin abzulassen. Sobald der gesamte Urin abgelassen ist, wird der Katheter entfernt. Es kann vorkommen, dass die Blase mehrmals am Tag entleert werden muss.

Katheter können aus Gummi, Latex, Silikon oder Polyvinyl (PVC) hergestellt sein. Sein Außendurchmesser wird häufig in Charrière (Charr.) oder in French (Fr) angegeben. Der Innendurchmesser (Kaliber) ist variabel. Ein Katheter zur intermittierenden Blasendrainage ist mit einem Gleitmittel, insbesondere einem Gleitgel oder einer Flüssigkeit, beschichtet, um das Einführen in die Blase zu erleichtern und die Harnröhre nicht zu verletzen. Einige Katheter sind mit einer gleitfähigen Wasserlösung beschichtet, die als "hydrophil" bezeichnet wird. Andere weisen kein Gleitmittel in der Verpackung oder auf dem Katheter auf, so dass der Benutzer das Gleitmittel selbst hinzufügen muss. Einige Katheter sind mit einer Schutzhülle überzogen, so dass der Katheter nicht direkt berührt wird. Bei anderen Kathetern muss ein in der Packung enthaltenes Wasserpäckchen aufgebrochen werden, welches den Katheter umhüllt. Alle Katheter sind Einwegkatheter, die nur einmal verwendet werden. Die meisten Katheter sind etwa 16 Zoll (bzw. 40,64cm) lang und haben eine gerade oder gebogene Spitze.

Kann sich die Blase nicht entleeren, so führt dies zu einer Reihe von Problemen. Beispielsweise kann die Blase überdehnt werden, was zu dauerhaften Schäden am Blasenmuskel und sogar zu Nierenschäden führen kann. Wenn der Urin über längere Zeit in der Blase verbleibt, kann dies des Weiteren zu einer Blasenentzündung führen. Ist man nicht in der Lage zu urinieren, so kann dies zu Schmerzen, Urinverlust, Harndrang, häufigem Wasserlassen und nächtlichem Harndrang führen.

### Zusammenfassung der Erfindung

Die vorliegende Erfindung betrifft eine Spritze die bei der Katheterisierung und insbesondere der Selbstkatheterisierung eingesetzt wird. Für Katheter verschiedener Kathetergrößen müssen bisher eigens jeweils geeignet dimensionierte Spritzen verwendet werden, um den jeweiligen Katheter an einer dafür passenden Spritze zu befestigen. Ein Beispiel für den Stand der Technik ist ES 27 122 U.

Vor diesem Hintergrund liegt der Erfindung nun die Aufgabe zugrunde, eine verbesserte Spritzenvorrichtung bereitzustellen.

Erfindungsgemäß wird diese Aufgabe durch eine Spritzenvorrichtung mit den Merkmalen des Schutzanspruchs 1 gelöst.

Es wird eine Spritzenvorrichtung bereitgestellt, die zur Injektion einer Flüssigkeit, insbesondere eines flüssigen Medikaments, oder zum Abnehmen einer Flüssigkeit dient, wobei die Spritzenvorrichtung aufweist:
ein Spritzengehäuse mit einem Spritzendeckel, wobei der Spritzendeckel einstückig mit einem trichterförmigen Katheter-Verbindungsadapter ausgebildet ist,
wobei der trichterförmige Katheter-Verbindungsadapter sich in Längsrichtung der Spritzenvorrichtung von einem inneren, dem Spritzengehäuse zugewandten Ende zu einem äußeren, dem Spritzengehäuse abgewandten Ende hin verjüngt,
wobei die Außenkontur des trichterförmigen Katheter-Verbindungsadapters in Längsrichtung abgestuft mit mehreren Stufenabschnitten und/oder stufenlos ausgebildet ist zum Befestigen von Kathetern mit unterschiedlichen Befestigungs-Innendurchmessern.

Weitere Aspekte und Ausführungsformen der Erfindung sind in den abhängigen Ansprüchen offenbart und können der folgenden Beschreibung, den Figuren und Beispielen entnommen werden, ohne darauf beschränkt zu sein.

Gemäß einer Ausführungsform der Erfindung ist bzw. sind wenigstens ein Stufenabschnitt oder mehrere Stufenabschnitte des trichterförmigen Katheter-Verbindungsadapters jeweils zylindrisch ausbildet.

In einer weiteren Ausführungsform der Erfindung ist wenigstens ein Stufenabschnitt oder mehrere Stufenabschnitte des trichterförmigen Katheter-Verbindungsadapters konisch ausgebildet und verjüngen sich in Längsrichtung der Spritzenvorrichtung von innen nach außen konisch.

In einem nicht erfindungsgemäßen Beispiel ist eine Außenkante des wenigstens einen, der mehreren oder aller Stufenabschnitte zusätzlich als abgerundete Außenkante ausgebildet.

Gemäß einer Ausführungsform der Erfindung weist wenigstens ein Stufenabschnitt oder mehrere Stufenabschnitte des trichterförmigen Katheter-Verbindungsadapters einen ersten oder in Längsrichtung der Spritzenvorrichtung inneren, zylindrisch ausgebildeten Abschnitt und einen daran anschließenden zweiten oder äußeren, konisch ausgebildeten Abschnitt auf, wobei der zweite oder äußere, konisch ausgebildete Abschnitt sich dabei in Längsrichtung der Spritzenvorrichtung von innen nach außen konisch verjüngt.

In einer Ausführungsform der Erfindung weist bzw. weisen wenigstens ein Stufenabschnitt oder mehrere Stufenabschnitte des trichterförmigen Katheter-Verbindungsadapters einen ersten oder in Längsrichtung der Spritzenvorrichtung inneren, konischen ausgebildeten Abschnitt und einen daran anschließenden zweiten oder äußeren, konisch ausgebildeten Abschnitt auf, wobei der erste und zweite konisch ausgebildete Abschnitt sich jeweils in Längsrichtung der Spritzenvorrichtung von innen nach außen konisch verjüngen, wobei der zweite oder äußere, konisch ausgebildete Abschnitt sich dabei stärker in Längsrichtung der Spritzenvorrichtung von innen nach außen konisch verjüngt als der erste, konisch ausgebildete Abschnitt.

In einer weiteren Ausführungsform der Erfindung ist eine Außenkante wenigstens eines Abschnitts des wenigstens einen Stufenabschnitts oder der mehreren Stufenabschnitte zusätzlich abgerundet oder als abgerundete Außenkante ausgebildet.

In einer anderen Ausführungsform der Erfindung sind alle Stufenabschnitte des trichterförmigen Katheter-Verbindungsadapters nach außen gewölbt ausgebildet.

Gemäß einer Ausführungsform der Erfindung sind alle Stufenabschnitte des trichterförmigen Katheter-Verbindungsadapters nach außen gewölbt ausgebildet und der Übergang zwischen allen Stufenabschnitten ist zusätzlich gerundet vorgesehen zur Ausbildung einer wellenförmigen oder rippenförmigen Außenkontur des trichterförmigen Katheter-Verbindungsadapters.

In einer Ausführungsform der Erfindung sind die wenigstens zwei oder alle Stufenabschnitte des trichterförmigen Katheter-Verbindungsadapters beispielsweise ballig, halbrund oder als abgerundete Rippe usw. nach außen gewölbt ausgebildet.

In einem nicht erfindungsgemäßen Beispiel ist die Außenkontur des trichterförmigen Katheter-Verbindungsadapters von seinem inneren, dem Spritzengehäuse zugewandten Ende zu einem äußeren, dem Spritzengehäuse abgewandten Ende, stufenlos, insbesondere glatt, ausgebildet. Der trichterförmige Katheter-Verbindungsadapter und/oder der Spritzendeckel kann bzw. können an ihrer Außenseite mit einem Greiferabschnitt ausgebildet sein, welcher beispielsweise zylindrisch ausgebildet ist, und vorzugsweise eine Länge von wenigstens 2mm aufweist. Der Greiferabschnitt hat den Vorteil, dass er durch eine Greifereinrichtung, beispielsweise eines entsprechenden Roboters, ergriffen und die Spritzenvorrichtung so problemlos transportiert werden kann. Der Übergang zwischen dem Greiferabschnitt und dem Spritzendeckel ist dabei beispielsweise zusätzlich gerundet, um das Desinfizieren oder Sterilisieren der Spritzenvorrichtung weiter zu vereinfachen. Gleiches gilt, wenn kein Greiferabschnitt vorgesehen ist. In diesem Fall kann der Übergang zwischen dem trichterförmigen Katheter-Verbindungsadapter und dem Spritzendeckel gerundet ausgebildet sein, um das Desinfizieren oder Sterilisieren der Spritzenvorrichtung weiter zu vereinfachen. Dies gilt bei allen Ausführungsformen der Erfindung.

In noch einer weiteren Ausführungsform der Erfindung weist der trichterförmige Katheter-Verbindungsadapter einen zusätzlichen äußersten Endabschnitt bzw. End-und Befestigungsabschnitt auf, welcher zum Befestigen, insbesondere dichten befestigen, eines Anschlusselements, z.B. einer Injektions-Nadel oder eines Verschlusselements, insbesondere einer Verschlusskappe, an dem äußeren Ende des trichterförmigen Katheter-Verbindungsadapters ausgebildet ist. Der äußerste Endabschnitt bzw. End- und Befestigungsabschnitt ist in einer Ausführungsform dabei vorzugsweise zylindrisch und/oder konisch und insbesondere als Luer-Konus ausgebildet.

Gemäß einer Ausführungsform der Erfindung ist ein äußerster Stufenabschnitt des trichterförmigen Katheter-Verbindungsadapters zum Befestigen, insbesondere dichten Befestigen, eines Katheters zusätzlich zum Befestigen, insbesondere dichten Befestigen, eines Anschlusselements, wie z.B. eines Verschlusselements, insbesondere einer Verschlusskappe, oder einer Injektions-Nadel usw., ausgebildet. Besonders bevorzugt ist der äußerster Stufenabschnitt des trichterförmigen Katheter-Verbindungsadapters dabei zum Befestigen eines Katheters und zum zusätzlichen Befestigen eines Anschlusselements, z.B. in Form der Injektions-Nadel oder eines Verschlusselements, z.B. in Form einer Verschlusskappe usw., zylindrisch und/oder konisch, insbesondere als Luer-Konus, ausgebildet.

In einer Ausführungsform der Erfindung ist der äußerste Endabschnitt an die Dimensionierung des Anschlusselements, z.B. Verschlusselements, Injektions-Nadel usw., angepasst, wobei der äußerste Endabschnitt des trichterförmigen Katheter-Verbindungsadapters zylindrisch und/oder konisch, insbesondere als Luer-Konus, ausgebildet ist und seine Länge und sein Außendurchmesser an eine Aufnahmelänge und einen Aufnahme-Innendurchmesser einer Aufnahme eines Anschlusselements, z.B. einer Verschlusskappe als Beispiel für ein Verschlusselement oder einer Injektions-Nadel angepasst ist zum Verschließen und insbesondere dichten Verschließen des äußeren Endes des trichterförmigen Katheter-Verbindungsadapters mit der Verschlusskappe bzw. zum dichten Anschließen der Injektions-Nadel. Das Anschlusselements, wie das Verschlusselement und insbesondere die Verschlusskappe sowie die Injektions-Nadel, liegt dabei dicht an zumindest einem umlaufenden Abschnitt des trichterförmigen Katheter-Verbindungsadapters an.

Wie zuvor ausgeführt, weist in einer Ausführungsform der Erfindung, der trichterförmige Katheter-Verbindungsadapter und/oder der Spritzendeckel einen Greiferabschnitt an ihrer Außenseite auf. Der Greiferabschnitt weist vorzugsweise eine Länge von wenigstens 2mm, um durch eine Greifereinrichtung geeignet ergriffen werden zu können. Des Greiferbaschnitt kann beispielsweise zylindrisch ausgebildet sein oder eine andere für eine Greifereinrichtung zum Ergreifen des Greiferabschnitts geeignete Form aufweisen.

In einer anderen Ausführungsform der Erfindung weist das Spritzengehäuse eine Durchgangsöffnung auf, die sich durch den Spritzendeckel mit dem trichterförmigen Katheter-Verbindungsadapter bis zu dem äußeren Ende hindurch erstreckt, wobei die Durchgangsöffnung beispielsweise zylindrisch und/oder trichterförmig ausgebildet ist.

In einer weiteren Ausführungsform der Erfindung weist die Spritzenvorrichtung eine Gesamtlänge von weniger als 21cm und insbesondere höchstens 20,5cm auf, wenn die Kolbenanordnung in eine Endposition ausgezogen ist, in welcher die Spritzenvorrichtung ein maximales, vorbestimmtes Spritzenvolumen aufweist, beispielsweise ein Spritzenvolumen in einem Bereich von vorzugsweise 1ml bis zu 20ml und besonders bevorzugt von 5ml bis 50ml.

Gemäß einer Ausführungsform der Erfindung ist das Spritzengehäuse mit dem Spritzendeckel und dem trichterförmigen Katheter-Verbindungsadapters einstückig aus Kunststoff, Metall und/oder Glas ausgebildet und insbesondere als Kunststoffspritzgussteil. Das Kunststoffspritzgussteil ist insbesondere ein Einkomponenten-Spritzgussteil oder wenigstens ein Zwei-Komponenten-Spritzgussteil oder ein Dreikomponenten-Spritzgussteil.

In einer Ausführungsform der Erfindung ist die Spritzenvorrichtung, insbesondere eine 1ml, 5ml, 10ml, 20ml, 30ml, 40ml oder 50ml Spritzenvorrichtung, wobei die Spritzenvorrichtung mit einem flüssigen Medikament, insbesondere mit Oxybutyninhydrochlorid vorgefüllt und mit einem Verschlusselement verschlossen ist.

### INHALTSANGABE DER ZEICHNUNG

Die vorliegende Erfindung wird nachfolgend anhand der in den schematischen Figuren der Zeichnungen angegebenen Ausführungsbeispiele näher erläutert. Es zeigen dabei:
- Fig. 1a-1c: schematische Ansichten verschiedener Positionen eines Patienten bei der Selbstkatheterisierung;
- Fig. 2a: eine Seitenansicht einer Spritzenvorrichtung mit einem trichterförmigen und zusätzlich abgestuften Katheter-Verbindungsadapter gemäß einem nicht erfindungsgemäßen Beispiel;
- Fig. 2b: eine Seitenansicht einer weiteren Spritzenvorrichtung mit einem trichterförmigen und zusätzlich abgestuften Katheter-Verbindungsadapter gemäß einem nicht erfindungsgemäßen Beispiel;
- Fig. 3a: eine schematische Ansicht eines nicht erfindungsgemäßen Beispiels eines trichterförmigen Katheter-Verbindungsadapters der erfindungsgemäßen Spritzenvorrichtung;
- Fig. 3b: eine schematische Ansicht eines weiteren trichterförmigen Katheter-Verbindungsadapters der erfindungsgemäßen Spritzenvorrichtung;
- Fig. 3c: eine Tabelle mit den Abmessungen des trichterförmigen Katheter-Verbindungsadapters gemäß Fig. 3a;
- Fig. 3d: eine schematische Ansicht eines anderen, stufenlosen trichterförmigen Katheter-Verbindungsadapters eines nicht erfindungsgemäßen Beispiels;
- Fig. 3e: eine schematische Ansicht eines weiteren Beispiels eines stufenlosen trichterförmigen Katheter-Verbindungsadapters einer Spritzenvorrichtung;
- Fig. 3f: eine schematische Ansicht eines Beispiels eines stufenlosen trichterförmigen Katheter-Verbindungsadapters einer Spritzenvorrichtung;
- Fig. 4: eine Perspektivansicht eines Schalensystems für Spritzenvorrichtungen der Fa. Kaatimex Ltd;
- Fig. 5: einen Ausschnitt eines Beispiels einer nicht erfindungsgemäßen Spritzenvorrichtung in einer Schnittansicht mit einem trichterförmigen Katheter-Verbindungsadapter und einem daran befestigten Katheter;
- Fig. 6: einen weiteren Ausschnitt eines Beispiels einer nicht erfindungsgemäßen Spritzenvorrichtung in einer Schnittansicht mit einem trichterförmigen Katheter-Verbindungsadapter und einem daran befestigten Katheter;
- Fig. 7: einen Ausschnitt eines anderen Beispiels einer nicht erfindungsgemäßen Spritzenvorrichtung in einer Schnittansicht mit einem trichterförmigen Katheter-Verbindungsadapter und einem daran befestigten Katheter;
- Fig. 8: einen Ausschnitt eines weiteren Beispiels einer nicht erfindungsgemäßen Spritzenvorrichtung in einer Schnittansicht mit einem trichterförmigen Katheter-Verbindungsadapter und einem daran befestigten Katheter;
- Fig. 9: einen Ausschnitt einer Ausführungsform einer erfindungsgemäßen Spritzenvorrichtung in einer Schnittansicht mit einem trichterförmigen Katheter-Verbindungsadapter und einem daran befestigten Katheter; und
- Fig. 10: einen vergrößerten Ausschnitt der erfindungsgemäßen Spritzenvorrichtung gemäß Fig. 9.

Die beigefügten Zeichnungen sollen Ausführungsformen der vorliegenden Erfindung veranschaulichen und ein weiteres Verständnis derselben vermitteln. In Verbindung mit der Beschreibung dienen sie zur Erläuterung der Konzepte und Prinzipien der Erfindung. Weitere Ausführungsformen und viele der genannten Vorteile lassen sich aus den Zeichnungen ableiten. Die Elemente der Zeichnungen sind nicht notwendigerweise maßstabsgetreu zueinander gezeigt.

Soweit nicht anders definiert, weisen die hier verwendeten technischen und wissenschaftlichen Begriffe die gleichen Bedeutungen auf, wie sie von einem Fachmann auf dem Gebiet der vorliegenden Erfindung allgemein verstanden werden.

In den Figuren der Zeichnung sind gleiche, funktionsgleiche und gleich wirkende Elemente, Merkmale und Komponenten - sofern nichts anderes ausgeführt ist - jeweils mit denselben Bezugszeichen versehen.

### BESCHREIBUNG VON AUSFÜHRUNGSBEISPIELEN

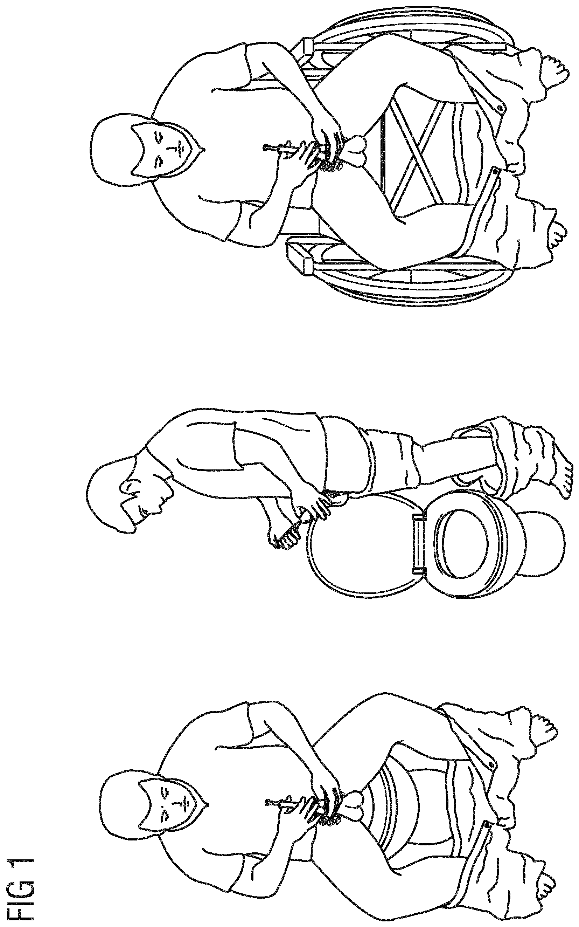

In den Fig. 1a, 1b und 1c sind verschiedene Positionen gezeigt, die man für den Katheterismus ausprobieren kann. So kann die Katheterisierung, wie in Fig. 1a gezeigt, auf der Toilette sitzend oder, wie in Fig. 1b gezeigt, im Stehen erfolgen. Ebenso kann die Katheterisierung, im Falle eines Rollstuhlfahrers, auch direkt im Rollstuhl sitzend durchgeführt werden, wie in Fig. 1c dargestellt ist.

Die Selbstkatheterisierung erfolgt dabei indem zunächst die Hände mit Wasser und Seife, z. B. anti-bakteriell wirkender Seife, gewaschen werden. Des Weiteren wird die Penisspitze mit Wasser und Seife gewaschen und anschließend die Öffnung an der Penisspitze abgewischt. Dann wird der Penis unterhalb der Eichel mit der nicht dominanten Hand gehalten und nach oben aufgerichtet. Wenn der Penis aufrecht gehalten wird, lässt sich der Katheter mit der anderen, dominanten Hand leicht einführen. Der Katheter wird langsam in die Harnröhrenöffnung eingeführt, ohne das glatte Ende des Katheters zu berühren. Der Katheter sollte, wenn der Urin zu fließen beginnt, 1-2 Zentimeter weiter vorgeschoben werden. Der Urin sollte abgelassen werden, bis der Urinfluss aufhört. Des Weiteren sollte ein Katheterismus nicht ausgelassen werden, da das Entleeren der Blase wichtig ist. Die Katheterisierung wird alle 6 Stunden und vor dem Schlafengehen empfohlen.

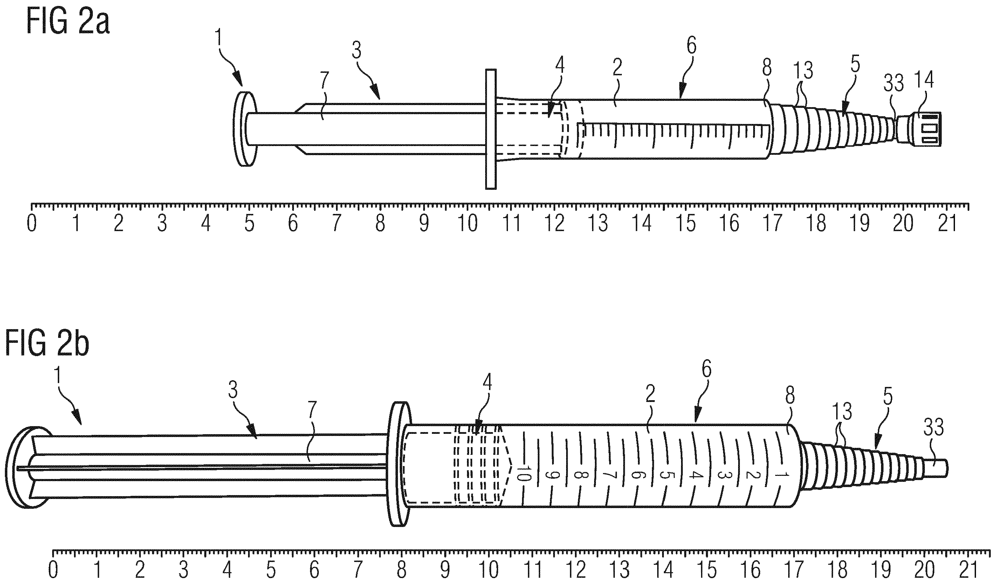

Anhand der nachfolgenden Ausführungsbeispiele in den Fig. 2a, 2b bis 10 wird die vorliegende Erfindung erläutert, welche auf eine Spritzenvorrichtung 1 gerichtet ist. Die Spritzenvorrichtung 1 weist einen Hohlzylinder 2 auf, der mit einer Kolbenanordnung 3 mit wenigstens einem verschiebbaren Kolben 4 und mit einem integrierten, trichterförmigen Katheter-Verbindungsadapter 5 an der Spitze ausgestattet ist. Der trichterförmige Katheter-Verbindungsadapter 5 der erfindungsgemäßen Spritzenvorrichtung 1 ist derart ausgebildet, dass er beispielsweise zum Anschließen geeignet ist von:
- urologischen Standardkathetern, wie zum Beispiel
   o Klarer Kunststoff (PVC)
   o Roter Gummi (Latex)
   o Hydrophile Katheter
   o Katheter mit gebogener Spitze (Coudé)
   o Geschlossene Katheter-Sets
   o Katheter mit Schutzhülse
- Standardgrößen, z. B. normalerweise von 18 bis 34 French oder entsprechend von 18 bis 34 Charrière,
- und unabhängig von der verwendeten Länge
- und unabhängig von dem Katheterhersteller, d. h. passend für alle verfügbaren Kathetersysteme, ohne dass ein geeigneter separater Katheter-Verbindungsadapter auf die Spitze einer Standardspritze aufgesetzt werden muss, bevor die Spritze an den Katheter angeschlossen wird.

In den Fig. 2a und 2b ist jeweils eine Abbildung einer Ausführungsform einer erfindungsgemäßen Spritzenvorrichtung 1 gezeigt.

Die Spritzenvorrichtung 1 weist dabei ein Spritzengehäuse 6 mit einem Hohlzylinder 2 und einer in dem Hohlzylinder 2 beweglich aufgenommenen Kolbenanordnung 3 auf. Die Kolbenanordnung 3, kann wie in den Fig. 2a und 2b gezeigt ist, z. B. aus einem Kolben 4 mit einer Kolbenstange 7 bestehen.

Die Erfindung ist jedoch auf diese spezielle Ausgestaltung nicht beschränkt. So kann die Kolbenanordnung 3 auch wenigstens einen zusätzlichen Kolben (nicht dargestellt) aufweisen, der den Hohlzylinder 2 in zwei Kammern unterteilt usw., je nach Funktion und Einsatzzweck.

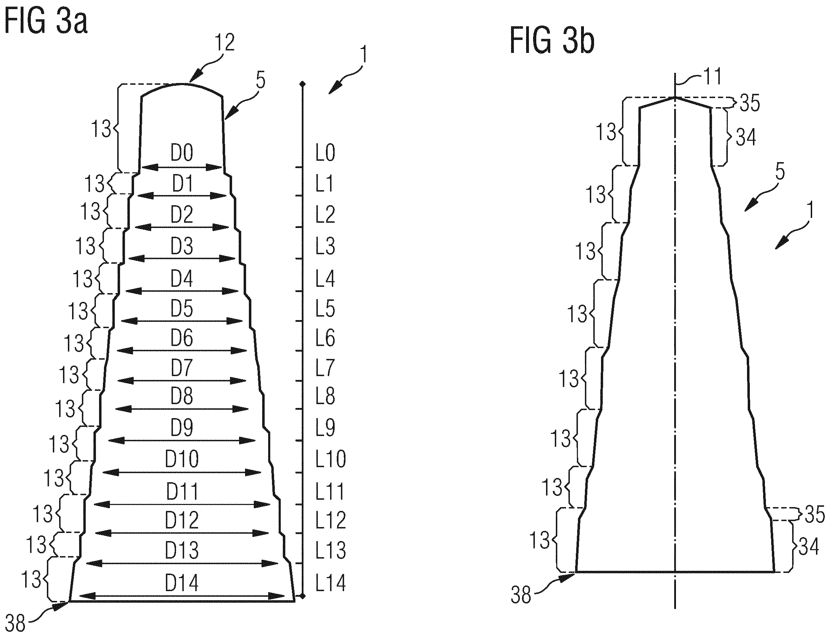

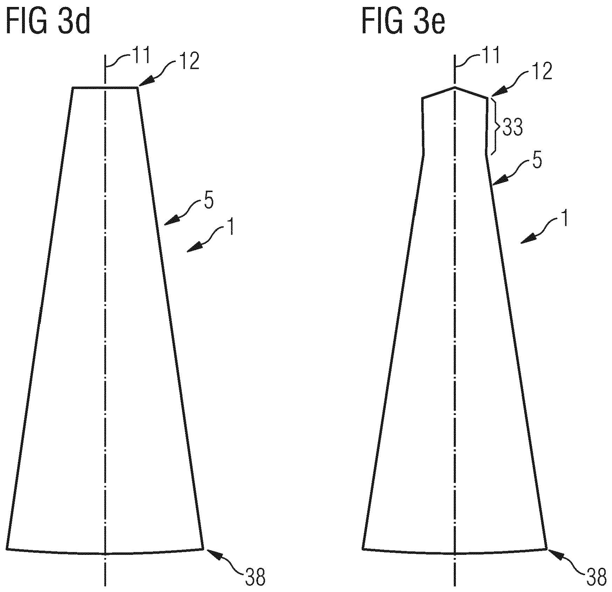

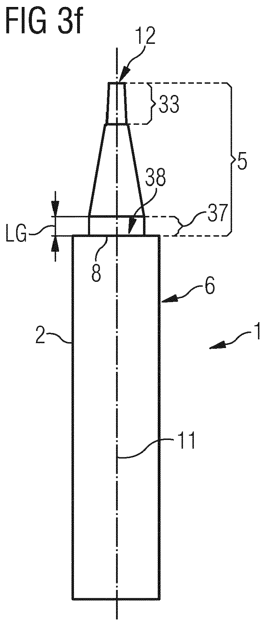

Das Spritzgehäuse 6 weist ein erstes offenes Ende auf, in welches die Kolbenanordnung 3 eingeführt ist, und ein zweites Ende, das einen Spritzendeckel 8 aufweist. An dem Spritzendeckel 8 ist dabei einstückig oder integral ein trichterförmiger Katheter-Verbindungsadapter 5 ausgebildet, der sich in Längsrichtung des Spritzengehäuses 6 nach außen verjüngt. Der trichterförmige Katheter-Verbindungsadapter 5 kann dabei beispielsweise eine abgestufte Außenkontur aufweisen, wie in Fig. 2a und 2b und den nachfolgenden Fig. 3a, 3b, 3c und 5-10 gezeigt ist. Ebenso kann der trichterförmige Katheter-Verbindungsadapter 5 auch eine glatte, insbesondere eine glatte konische oder glatte kegelstumpfförmige Außenkontur aufweisen, wie in dem Ausführungsbeispiel in nachfolgenden Fig. 3d und 3e und 3f beispielhaft gezeigt ist.

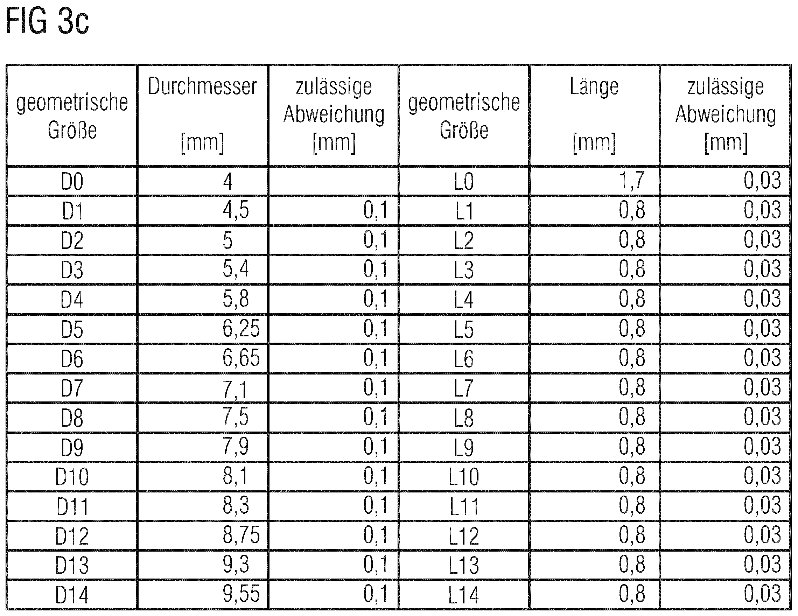

Darüber hinaus, kann ein trichterförmiger Katheter-Verbindungsadapter 5 aber auch beispielsweise einen oder mehrere Stufenabschnitte, wie in den Fig. 3a, 3b, 3c und 5-10 aufweisen und ansonsten eine stufenlose oder glatte Außenkontur, wie in den Fig. 3c und 3d, 3e und 3f, je nach Funktion und Einsatzzweck. Die Ausführungsbeispiele in den Fig. 2a, 2b, 3a-3f und 5-10 können beliebig miteinander kombiniert werden, insbesondere deren Außenkontur mit Stufenabschnitten, wie in den Fig. 2a, 2b, 3a-c und 5-10, sowie die stufenlose konische oder glatte konische Außenkontur in den Fig. 3d, 3e und 3f.

Der trichterförmige Katheter-Verbindungsadapter 5 ist derart ausgebildet, dass er mit einer Vielzahl von Kathetern mit einem jeweils unterschiedlichen Befestigungs-Innendurchmesser 36 verbunden werden kann. Der Befestigungs-Innendurchmesser 36 ist dabei der Katheter-Innendurchmesser am Ende des Katheters 9, mit dem der Katheter 9 auf den Katheter-Verbindungsadapter 5 aufgeschoben und so an ihm befestigt wird. Je nach Kathetergröße des Katheters, z. B. in einem Bereich von 18 bis 34 French oder entsprechend von 18 bis 34 Charrière, können Katheter verschiedene Befestigungs-Innendurchmesser 36 aufweisen. Dem entsprechend muss für jede Kathetergröße und deren Befestigungs-Innendurchmesser 36, bisher eine eigene passende Spritzenvorrichtung zum Anschließen des Katheters bereitgestellt werden.

Durch die erfindungsgemäße Spritzenvorrichtung 1 mit ihrem einstückig daran ausgebildeten trichterförmigen Katheter-Verbindungsadapter 5 können jedoch unterschiedliche Kathetergrößen, z. B. Katheter 9 mit einer Kathetergröße in einem Bereich von 18 bis 34 French oder entsprechend von 18 bis 34 Charrière, an dem trichterförmigen Katheter-Verbindungsadapter 5 befestigt werden. Dadurch muss nicht für jede Kathetergröße eine eigens dafür passende Spritzenvorrichtung bereitgehalten werden. Stattdessen lassen sich an der erfindungsgemäßen Spritzenvorrichtung 1 aufgrund ihres trichterförmigen Katheter-Verbindungsadapters 5 diverse Katheter 9 unterschiedlicher Kathetergrößen mit entsprechend unterschiedlichen Befestigungs-Innendurchmessern 36 sehr leicht befestigen. Da der trichterförmige Katheter-Verbindungsadapter 5 als ein Teil oder einstückig mit der Spritzenvorrichtung 1 ausgebildet ist, hat er des Weiteren den Vorteil, dass er nicht als separates Bauteil an der Spritzenvorrichtung 1 erst befestigt werden muss, bevor der Katheter 9 anschließend mit der Spritzenvorrichtung 1 verbunden werden kann. Die einteilige oder einstückige Ausbildung der Spritzenvorrichtung 1 mit dem trichterförmigen Katheter-Verbindungsadapter 5 ist dadurch wesentlich leichter in der Produktion zu sterilisieren, deutlich hygienischer in der Handhabung und erlaubt zudem eine wesentlich einfachere Handhabung für einen Benutzer, da der Benutzer seinen Katheter 9 lediglich auf den trichterförmigen Katheter-Verbindungsadapter 5 der Spritzenvorrichtung 1 aufschieben muss, bis er geeignet fest auf dem trichterförmigen Katheter-Verbindungsadapter 5 aufsitzt.

Der trichterförmige Katheter-Verbindungsadapter 5 der erfindungsgemäßen Spritzenvorrichtung 1 verjüngt sich in Längsrichtung des Spritzengehäuses 6 nach außen. Dabei kann der trichterförmige Katheter-Verbindungsadapter 5 sich gänzlich oder abschnittsweise stufenlose und/oder ebenso abgestuft bzw. stufenweise in Längsrichtung des Spritzengehäuses 6 nach außen verjüngen, zum Verbinden mit unterschiedlichen Kathetern 9, insbesondere Kathetern die jeweils einen unterschiedliche Befestigungs-Innendurchmesser 36 aufweisen, wie im Folgenden noch näher erläutert wird.

Der trichterförmige Katheter-Verbindungsadapter 5 kann ebenso als trichterförmiger Adapter bezeichnet werden. Dies gilt für alle hierin beschriebenen Ausführungsformen der Erfindung.

Der trichterförmige Katheter-Verbindungsadapter 5 ist dabei mit dem Spritzengehäuse 6 und dessen Hohlzylinder 2 mit dem Spritzendeckel 8 einstückig oder integral ausgebildet, beispielsweise aus Kunststoff, Glas und/oder Metall.

Das Spritzengehäuse 6 weist des Weiteren eine Durchgangsöffnung 10 auf, die sich durch den Spritzendeckel 8 mit dem trichterförmigen Katheter-Verbindungsadapter 5 hindurch erstreckt und insbesondere bis zu dem äußeren Ende 12 des Katheter-Verbindungsadapters 5. Dadurch kann mittels der Spritzvorrichtung 1 ein in dem Hohlzylinder 2 des Spritzengehäuses 6 aufgenommenes, flüssiges Medikament durch die Durchgangsöffnung 10 des Spritzendeckels 8 und seines daran ausgebildeten trichterförmigen Katheter-Verbindungsadapters 5 ausgegeben bzw. verabreicht oder umgekehrt eine Flüssigkeit, z. B. eine Körperflüssigkeit oder ein flüssiges Medikament usw., aufgenommen und in den Hohlzylinder 2 eingesaugt werden.

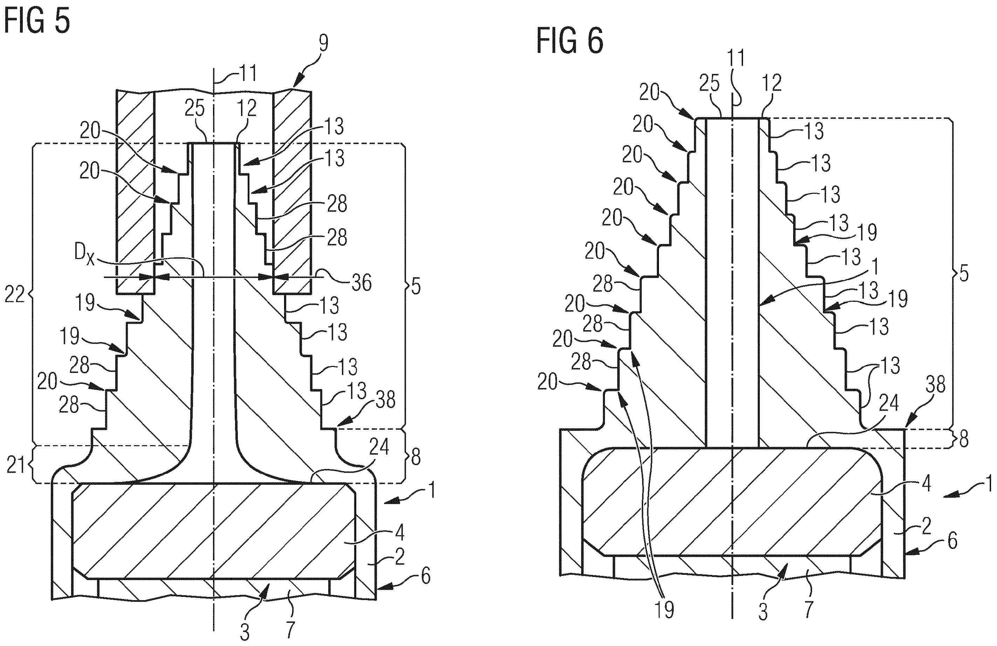

In den nachfolgenden Fig. 5 bis 10 sind neben verschiedenen Außenkonturen des trichterförmigen Katheter-Verbindungsadapters 5 auch verschiedene Ausgestaltungen der Durchgangsöffnung 10 beschrieben, die bei der Spritzenvorrichtung 1 in den Fig. 2a und 2b beispielsweise vorgesehen werden kann, ohne darauf beschränkt zu sein.

Wie in den Fig. 2a und 2b gezeigt ist, verjüngt sich der trichterförmige Katheter-Verbindungsadapter 5 in Längsrichtung, d. h. entlang der Längsachse 11 der Spritzenvorrichtung 1 und ihres Spritzengehäuses 6, nach außen oder mit anderen Worten, von seinem inneren oder dem Spritzengehäuse 6 zugewandten Ende zu seinem äußeren oder dem Spritzengehäuse 6 abgewandten Ende 12 hin, beispielsweise stufenweise oder in Stufenabschnitten 13, wie z. B. den Fig. 2a und 2b und nachfolgenden Fig. 3a, 3b, 3c und 5-10, oder stufenlos, wie in den Beispielen in nachfolgenden Fig. 3d, 3e und 3f gezeigt. Das äußere Ende 12 des trichterförmigen Katheter-Verbindungsadapters 5 zeigt von dem Spritzendeckel 8 nach außen in Längsrichtung und damit weg von dem Spritzengehäuse 6.

Gemäß der Erfindung ist der trichterförmige Katheter-Verbindungsadapter 5 dabei einstückig oder integral mit dem Spritzendeckel 8 und dem Hohlzylinder 2 des Spritzengehäuses 6 ausgebildet z. B. als Spritzgussteil. Das Spritzengehäuse 6 mit dem Hohlzylinder 2 und seinem Spritzendeckel 8 mit dem trichterförmigen Katheter-Verbindungsadapter 5 kann dabei beispielsweise ein Einkomponenten-Spritzgussteil sein, bei welcher das Spritzengehäuse 6 mit seinem Hohlzylinder 2 und der Spritzendeckel 8 mit dem trichterförmigen Katheter-Verbindungsadapter 5 aus demselben Material, hier einem Kunststoff, hergestellt sind. Ebenso können das Spritzengehäuse 6, der Spritzendeckel 8 und der trichterförmige Katheter-Verbindungsadapter 5 auch ein Mehrkomponenten-Spritzgussteil sein, bei welchem beispielsweise das Spritzengehäuse 6 mit seinem Hohlzylinder 2, der Spritzendeckel 8 und/oder der trichterförmige Katheter-Verbindungsadapter 5 jeweils aus einem unterschiedlichen Kunststoff hergestellt sind, je nach Funktion und Einsatzzweck. So kann das Spritzengehäuse 6 mit dem Hohlzylinder 2, dem Spritzendeckel 8 und dem trichterförmigen Katheter-Verbindungsadapter 5 als Einkomponenten-, Zweikomponenten-, Dreikomponenten- oder Mehrkomponenten Spritzgussteil ausgebildet sein.

Beispielsweise kann als Kunststoff für das Spritzengehäuse 6 und genauer den Hohlzylinder 2, den Spritzendeckel 8 und/oder den trichterförmigen Katheter-Verbindungsadapter 5 Polypropylen (PP), Polyethylen (PE), Cycloolefin-Copolymer(e) (COC) und/oder Polypropylenharz oder ein anderes Kunststoffpolymer oder andere Kunststoffpolymere usw. vorgesehen werden. Dies gilt für alle Ausführungsformen der Erfindung.

Die Spritzenvorrichtung 1 und insbesondere das Spritzengehäuse 6, besonders sein Hohlzylinder 2, der Spritzendeckel 8 und/oder der trichterförmige Katheter-Verbindungsadapter 5 können sowohl aus wenigstens einem Kunststoff, wie z. B. PP, PE und/oder COC, aus Glas und/oder aus Metall hergestellt werden, je nach Funktion und Einsatzzweck.

Die erfindungsgemäße Spritzenvorrichtung 1 mit ihrem trichterförmigen Katheter-Verbindungsadapter 5 ist beispielsweise zum Anschluss von Standard-Kathetern 9 mit einer Kathetergröße von z. B. wenigstens 18 bis 34 French ausgebildet. Dies gilt für alle Ausführungsformen der Erfindung.

Dem entsprechend sind die Stufenabschnitte 13 des trichterförmigen Katheter-Verbindungsadapters 5 derart ausgebildet und dimensioniert, wie z. B. in nachfolgenden Fig. 3a, 3b und 3c gezeigt, dass Standardkatheter 9 mit einem jeweiligen Befestigungs-Innendurchmesser 36 für eine Kathetergröße von z. B. 18 bis 34 French, auf einen jeweiligen zugeordneten Stufenabschnitt 13 des trichterförmigen Katheter-Verbindungsadapters 5 aufgeschoben und daran befestigbar sind.

In den Ausführungsbeispielen in Fig. 2a und 2b ist jeweils eine Spritzenvorrichtung 1 mit einem einstückig daran ausgebildeten trichterförmigen Katheter-Verbindungsadapter 5 gezeigt, wobei der trichterförmige Katheter-Verbindungsadapter 5 sich dabei stufenweise oder in Stufenabschnitten 13 zu seinem äußeren Ende 12 hin verjüngt. Fig. 2a zeigt z. B. eine Standard-5-ml-Spritze und Fig. 2b z. B. eine Standard-10-ml Spritze. Im Gegensatz zu Fig. 2b ist der Katheter-Verbindungsadapter 5 der Spritzenvorrichtung 1 in Fig. 2a mit einem zusätzlichen Anschlusselement, z.B. einem Verschlusselement, hier z. B. einer Verschlusskappe 14 beispielsweise aus Gummi oder einem anderen geeigneten Material oder geeigneten Materialkombination, versehen.

Das Spritzengehäuse 6 mit seinem Hohlzylinder 2, dem Spritzendeckel 8 und dem trichterförmigen Katheter-Verbindungsadapter 5 ist dabei einstückig oder integral, z. B. als Spritzgussteil, ausgebildet. Der trichterförmige Katheter-Verbindungsadapter 5 kann dabei beispielsweise an seinem äußeren Ende 12 statt einen Stufenabschnitt als äußersten Befestigungsabschnitt für die kleinste Kathetergröße auch einen äußersten Endabschnitt 33 aufweisen, wie in Fig. 2a und 2b gezeigt ist, zum Befestigen beispielsweise eines Anschlusselements, z.B. eines Verschlusselements.

In Fig. 2a ist dabei als Anschlusselement, hier z.B. Verschlusselement z. B. die Verschlussklappe 14 an dem äußersten Endabschnitt 33 vorgesehen. Der äußerste Endabschnitt 33 ist als End- und Befestigungsabschnitt derart ausgebildet, dass ein Anschlusselement, hier z.B. ein Verschlusselement, wie z. B. die Verschlusskappe 14, an dem äußersten Endabschnitt 33 befestigbar, insbesondere aufsteckbar ist, um die Durchgangsöffnung 10 am äußeren Ende 12 des trichterförmigen Katheter-Verbindungsadapters 5 zu verschließen und vorzugsweise dicht zu verschließen, beispielsweise dicht zu umschließen. Dabei ist z. B. die Länge und der Außendurchmesser des äußersten Endabschnitts 33 an das daran zu befestigende Anschlusselement, hier z.B. das Verschlusselement, z. B. die Verschlusskappe 14, angepasst. Beispielsweise kann der äußerste Endabschnitt 33 insbesondere in den Fig. 2a und 2b als End- und Befestigungsabschnitt z.B. zylindrisch oder als Luer_Konus ausgebildet sein. Dies gilt für alle Ausführungsformen der Erfindung. Auf diese Weise kann das äußere Ende 12 der Spritzenvorrichtung 1 im Falle des Verschlusselements verschlossen und vorzugsweise zusätzlich abgedichtet werden bzw. im Falle eines Anschlusselements, wie einer Injektions-Nadel, diese an das äußere Ende 12 der spritzenvorrichtung 1 angeschlossen und insbesondere zusätzlich dicht angeschlossen werden. Dies hat den Vorteil, dass eine Verschmutzung oder Kontaminierung der Spritzenvorrichtung 1 über das äußere Ende 12 mittels des Verschlusselements, hier der Verschlusskappe, verhindert werden kann. Im Falle einer mit einem flüssigen Medikament vorgefüllten Spritzenvorrichtung 1, kann wirksam ein ungewolltes Austreten dieser Flüssigkeit aus der Spritzenvorrichtung 1 verhindert werden. Grundsätzlich kann aber auch statt eines äußeren Endabschnitts 33, wie in den Fig. 2a und 2b, auch ein äußerster Stufenabschnitt 13 für die Katheterbefestigung ebenfalls mit einem Anschlusselement, z.B. einem Verschlusselement, wie z. B. einer Verschlusskappe, zusätzlich verschließbar, insbesondere dicht verschließbar, bzw. zusätzlich anschließbar und insbesondere dicht anschließbar vorgesehen sein. Dies ist dann der Fall, wenn an dem äußersten Stufenabschnitt 13 für die Katheterbefestigung beispielsweise eine Injektions-Nadel als Anschlusselement befestigt und angeschlossen wird, vorzugsweise dicht angeschlossen wird. Dies gilt für alle Ausführungsformen der Erfindung. Des Weiteren kann die Spritzenvorrichtung 1 in den Fig. 2a und 2b, sowie den nachfolgenden Fig. 3a-3e neben einem zylindrischen End-und Befestigungsabschnitt 33 oder einem mit einem Luer-Konus ausgebildeten End-Und Befestigungsabschnitt 33 auch zusätzlich oder alternativ einen Greiferabschnitt 37 aufweisen, wie er mit Bezug auf die nachfolgende Fig. 3f gezeigt und beschrieben wird.

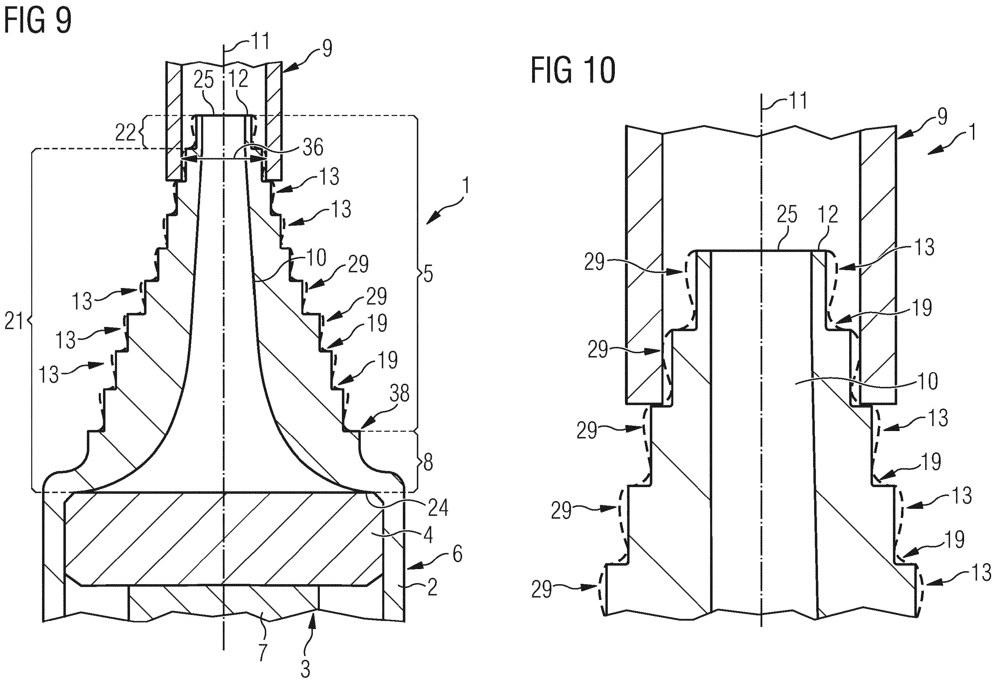

Beide in den Fig. 2a und 2b gezeigten Spritzenvorrichtungen 1 mit ihren trichterförmigen Katheter-Verbindungsadaptern 5 erfüllen vorzugsweise die Anforderungen für die Nesttechnologie bei der automatischen Vorfüllung von Standardspritzen mit einer Gesamtlänge von maximal 21,0 cm. In den Fig. 2a und 2b sind die beiden Spritzenvorrichtung 1 vorgefüllt und die Kolbenanordnung 3, hier aus Kolben 4 und Kolbenstange 7, in eine Endposition ausgezogen, in welcher die jeweilige Spritzenvorrichtung 1 auf das vorbestimmte maximale Spritzenvolumen von 5ml in Fig. 2a und 10ml in Fig. 2b vollgefüllt ist. Wie in Fig. 2a und 2b gezeigt ist, erfüllen die beiden Spritzenvorrichtungen 1 dabei die Anforderungen an die Nesttechnologie und übersteigen nicht die Gesamtlänge von 21cm.

Wie in den Beispielen in Fig. 2a und 2b gezeigt ist, ist der jeweilige trichterförmige Katheter-Verbindungsadapter 5 der Spritzenvorrichtungen 1 an seiner Außenseite zusätzlich abgestuft ausgebildet und weist eine abgestufte Außenkontur mit mehreren Stufenabschnitte 13 auf zur Aufnahme und Befestigung von verschiedenen, vorbestimmten Kathetergrößen. Der trichterförmige Katheter-Verbindungsadapter 5 mit seiner abgestuften Außenkontur aus mehreren Stufenabschnitten 13 verjüngt sich entlang der Längsachse 11 der Spritzenvorrichtung 1 und ihres Spritzengehäuses 6, von seinem inneren Ende zu seinem äußeren Ende 12.

Wie im Folgenden noch näher beschrieben wird, können die Stufenabschnitt 13 im einfachsten Fall zylindrisch ausgebildet sein. Wahlweise können die Übergänge zwischen den Stufenabschnitten 13 zusätzlich gerundet sein. Dies gilt für alle Ausführungsformen der Erfindung. Gerundete Übergänge haben den Vorteil, dass sie leichter desinfiziert werden können. Ebenso können die Außenkanten der Stufenabschnitte 13 wahlweise zusätzlich abgerundet sein. Dies gilt ebenfalls für alle Ausführungsformen der Erfindung. Die abgerundeten Kanten oder Außenkanten der Stufenabschnitte 13 haben den Vorteil, dass ein Katheter 9 noch leichter auf den jeweiligen Stufenabschnitt 13 des trichterförmigen Katheter-Verbindungsadapters 5 aufgeschoben werden kann.

In den Fig. 3a und 3b ist eine rein schematische und stark vereinfachte Darstellung der Außenkontur des trichterförmigen Katheter-Verbindungsadapters 5 gemäß einer Ausführungsform der erfindungsgemäßen Spritzenvorrichtung 1 gezeigt. Der trichterförmige Katheter-Verbindungsadapter 5 in Fig. 3a weist dabei eine größere Anzahl von Stufenabschnitten 13 als der trichterförmige Katheter-Verbindungsadapter 5 in Fig. 3b auf. Weitere Details, wie die Durchgangsöffnung des trichterförmigen Katheter-Verbindungsadapters 5 und die Verbindung zu der übrigen Spritzenvorrichtung 1, insbesondere deren Spritzendeckel und Spritzengehäuse, sind in den Fig. 3a und 3b aus Gründen der Übersichtlichkeit nicht dargestellt.

Jeder Stufenabschnitt 13 des trichterförmigen Katheter-Verbindungsadapters 5 in den Fig. 3a und 3b, weist dabei in Längsrichtung des trichterförmigen Katheter-Verbindungsadapters 5, einen ersten oder (in Längsrichtung) inneren konischen Abschnitt 34 und einen zweiten oder (in Längsrichtung) äußeren konischen Abschnitt 35 auf. Der zweite oder obere konische Abschnitt 35 ist dabei in Längsrichtung des trichterförmigen Katheter-Verbindungsadapters 5 kürzer als der erste oder untere konische Abschnitt 34 und außerdem ist der zweite oder obere konische Abschnitt 35 in Längsrichtung stärker konisch zu dem äußeren Ende 12 des Katheter-Verbindungsadapters 5 hin verjüngt ausgebildet als der erste oder untere konische Abschnitt 34, wie in Fig. 3a und 3b gezeigt ist.

Wie zuvor beispielhaft mit Bezug auf die Fig. 2a und 2b beschrieben wurde, kann der trichterförmige Katheter-Verbindungsadapter 5 in den Fig. 3a und 3b beispielsweise an seinem äußeren Ende 12 statt einen äußersten oder letzten Stufenabschnitt 13 als äußersten Befestigungsabschnitt für einen Katheter auch einen äußersten Endabschnitt zum Befestigen beispielsweise eines Anschlusselements, z.B. eines Verschlusselements, aufweisen, oder der äußerste Stufenabschnitt 13 kann selbst sowohl zur Befestigung eines Katheters sowie eines Anschlusselements, z.B. eines Verschlusselements, wie zuvor der Verschlusskappe 14 in den Fig. 2a und 2b, oder einer Injektions-Nadel (nicht dargestellt) vorgesehen werden. Wie in Fig. 3a gezeigt ist, ist der äußerste Stufenabschnitt 13 hierbei zylindrisch oder alternativ z.B. auch konisch (nicht dargestellt) und insbesondere als Luer Konus ausgebildet und beispielsweise verlängert ausgeführt, so dass z. B. eine Verschlusskappe als Anschluss- und Verschlusselement auf den äußersten Stufenabschnitt 13 aufgesteckt werden kann. Dies gilt für alle Ausführungsformen der Erfindung. Wie in nachfolgender Fig. 3f kann der erste Stufenabschnitt 13 des trichterförmige Verbindungsadapter 5 an seinem inneren Ende 38 des Verbindungsadapters 5 in den Fig. 3a und 3b auch alternativ als Greiferabschnitt 37 ausgebildet sein, wie mit Bezug auf Fig. 3f gezeigt und beschrieben wird. Der Greiferabschnitt 37 kann dabei wahlweise zusätzlich auch gleichzeitig als Stufenabschnitt zum Befestigen eines zugeordneten Katheters ausgebildet sein und dem entsprechend eine Doppelfunktion bereitstellen. Dies gilt für alle Ausführungsformen der Erfindung, wie sie insbesondere mit Bezug auf die Fig. 2a. 2b-10 beschrieben wird.

Ein Beispiel für die Abmessungen des in Fig. 3a gezeigten trichterförmigen Katheter-Verbindungsadapters 5 ist in der Tabelle in Fig. 3c aufgeführt. Der trichterförmige Katheter-Verbindungsadapter 5 in Fig. 3a weist beispielsweise fünfzehn Stufenabschnitte 13 oder Stufen auf. Die fünfzehn Stufenabschnitte 13 mit jeweils ihrem in Längsrichtung ersten oder unteren konischen Abschnitt 34 und dem zweiten oder oberen konischen Abschnitt 35, weisen dabei als geometrische Größe den (Außen-) Durchmesser D auf, beginnend mit dem größten (Außen-) Durchmesser *D14* des innersten Stufenabschnitts 13 und endend mit dem kleinsten (Außen-) Durchmesser D0 des äußersten Stufenabschnitts 13 am äußersten Ende 12 des Katheter-Verbindungsadapters 5. Mit anderen Worten, der an dem inneren Ende des trichterförmigen Katheter-Verbindungsadapters 5 in Längsrichtung der Spritzenvorrichtung 1 innerste Stufenabschnitt 13, der mit dem Spritzendeckel 8 und damit dem Spritzengehäuse 6 einstückig ausgebildet ist, weist den größten (Außen-) Durchmesser *D14* auf. Der an dem äußeren Ende 12 des trichterförmigen Katheter-Verbindungsadapters 5 in Längsrichtung fünfzehnte oder äußerste Stufenabschnitt 13 des trichterförmigen Katheter-Verbindungsadapters 5 weist wiederum den kleinsten (Außen-) Durchmesser *D0* auf.

Des Weiteren weisen die Stufenabschnitte 13 als geometrische Größe außerdem die Länge *L* bzw. Stufenhöhe auf. Die ersten vierzehn Stufenabschnitte 13 des trichterförmigen Katheter-Verbindungsadapters 5 mit ihren Längen *L14* bis *L1,* weisen jeweils z. B. dieselbe Länge bzw. Stufenhöhen auf nämlich z. B. *L1 = L2 = L3 =...L14 =* 0,8mm. Lediglich der fünfzehnte oder äußerste Stufenabschnitt 13 des trichterförmigen Katheter-Verbindungsadapters 5, welcher wahlweise zusätzlich zur Befestigung eines Anschlusselements, z.B. einer Injektions-Nadel oder eines Verschlusselements, wie die Verschlusskappe 14 in Fig. 2a, genutzt werden kann, weist eine Länge *L0* bzw. Stufenhöhe von z. B. 1,7mm und damit z. B. eine größere Länge bzw. Stufenhöhe als die anderen Stufenabschnitte 13 auf.

Die Erfindung ist jedoch auf eine Länge *L* bzw. Stufenhöhe der Stufenabschnitte 13 des trichterförmigen Katheter-Verbindungsadapters 5 in einem Bereich von 0,8mm bis 1,7mm nicht beschränkt. Die Länge *L* bzw. Stufenhöhe der Stufenabschnitte 13 des trichterförmigen Katheter-Verbindungsadapters 5 kann kleiner als 0,8mm und größer als 1,7mm sein, je nach Funktion und Einsatzzweck. Gleiches gilt für die Durchmessergrößen *D* und Anzahl der Stufenabschnitte 13 in Fig. 3a, und 3b. Die (Außen-) Durchmesser *D* der Stufenabschnitte 13 des trichterförmigen Katheter-Verbindungsadapters 5 sind entsprechend der anzuschließenden Katheter und ihrer Kathetergrößen und deren zugeordneten Befestigungs-Innendurchmesser gewählt, mit dem die Katheter auf die Stufenabschnitte 13 des Katheter-Verbindungsadapters 5 zum Befestigen aufgesteckt oder aufgeschoben werden. Die Abmessungen in Fig. 3a für die (Außen-) Durchmesser *D* sind lediglich beispielhaft. Des Weiteren können alle Stufenabschnitte 13 dieselbe Länge *L* oder wenigstens zwei Stufenabschnitte 13 unterschiedliche Längen *L* aufweisen, je nach Funktion und Einsatzzweck. Dies gilt für alle Ausführungsformen der Erfindung.

In den Fig. 3d, 3e und 3f ist jeweils ein weiteres Ausführungsbeispiel einer Spritzenvorrichtung 1 mit einem einstückig oder integral daran vorgesehenen, trichterförmigen Katheter-Verbindungsadapter 5 gezeigt. Der trichterförmige Katheter-Verbindungsadapter 5 der erfindungsgemäßen Spritzenvorrichtung 1 verjüngt sich dabei stufenlos und insbesondere konisch oder kegelstumpfförmige in Längsrichtung des Spritzengehäuses 6 nach außen, zum Verbinden mit unterschiedlichen Kathetergrößen, z. B. in einem Bereich von 18 bis 34 French oder entsprechend von 18 bis 34 Charrière, und dem entsprechend Kathetern mit einem unterschiedlichen Befestigungs-Innendurchmesser. Dabei kann, wie in Fig. 3e und 3f gezeigt ist, der trichterförmige Katheter-Verbindungsadapter 5 wahlweise zusätzlich einen äußersten, insbesondere zylindrischen oder wie in dem Beispiel in Fig. 3f konischen, End- und Befestigungsabschnitt 33 für die Befestigung eines Anschlusselements, z.B. eines Verschlusselements, z. B. einer Verschlusskappe 14, wie zuvor in den Fig. 2a und 2b aufweisen.

In dem Beispiel der Spritzvorrichtung 1 in Fig. 3f ist der konische End- und Befestigungsabschnitt 33 beispielsweise als Luer-Konus ausgebildet zum Befestigen einer dazu korrespondierenden (nicht dargestellte) Verschlusskappe oder eine (nicht dargestellten) Injektions-Spritze usw. mittels des Luer-Lock-Prinzips. Der konische End-und Befestigungsabschnitt 33 beispielsweise in Form eines Luer-Konus ist vorzugsweise derart dimensioniert, dass es zum abdichtendem Befestigen einer marktgeführten Standardlösung als Verschlusskappe von Fertigspritzen geeignet ist, z.B. Tip-Cap^{®}, die dank weichem anschmiegsamen Material (Gummi) einen wasserdichten Verschluss ermöglicht. Gleiches gilt für Injektions-Nadeln als einem weiteren Beispiel für ein Anschlusselement zum Anschließen an die erfindungsgemäße Spritzenvorrichtung.

Der stufenlose oder glatte, sich konisch nach außen verjüngende Abschnitt der Außenkontur, wie er insbesondere in den Fig. 3e und 3f gezeigt ist, geht vorzugsweise in den zylindrisch oder, wie in Fig. 3f gezeigt, konisch, insbesondere als Luer-Konus ausgebildeten äußersten End- und Befestigungsabschnitt 33 über. Der äußerste End-und Befestigungsabschnitt 33 ist vorzugsweise bezüglich seiner Länge und seines Außendurchmessers an eine Aufnahmelänge und einen Aufnahme-Innendurchmesser einer zugeordneten Verschlusskappe oder zugeordneten Injektions-Nadel und ihrer jeweiligen Aufnahme für das äußere Ende 12 des trichterförmigen Katheter-Verbindungsadapters 5 angepasst, so dass die Verschlusskappe den trichterförmigen Katheter-Verbindungsadapter 5 und sein äußeres Ende 12 verschließen und insbesondere dicht verschließen kann bzw. die Injektions-Nadel an dem trichterförmigen Katheter-Verbindungsadapter 5 und seinem äußeren Ende 12 angeschlossen und insbesondere dicht angeschlossen werden kann. Im Falle eines als Luer-Konus ausgebildeten End- und Befestigungsabschnitts 33, wie in dem Beispiel in Fig. 3f gezeigt, ist ein Anschlusselement, insbesondere ein Verschlusselement, z.B. eine Verschlusskappe, zu dem Luer-Konus korrespondierend ausgebildet, um mit diesem mittels des Luer-Lock Prinzips verbunden zu werden, insbesondere dicht verbunden zu werden. Auch andere Anschlusselemente wie z.B. Injektions-Nadeln usw. können dank der Standard-Dimensionen des Befestigungsabschnitts 33 auf die Spritzenvorrichtung aufgesetzt werden.

In dem Ausführungsbeispiel in Fig. 3f ist dabei ein Ausführungsbeispiel der erfindungsgemäßen Spritzenvorrichtung 1 in einer Vorderansicht gezeigt. Die Kolbenanordnung 3 und die Durchführungsöffnung 10 sind dabei in Fig. 3f im Gegensatz zu den Fig. 2a, 2b und 5-10 aus Gründen der Übersichtlichkeit nicht dargestellt.

Wie in Fig. 3f gezeigt, weist der trichterförmige Katheter-Verbindungsadapter 5 der Spritzenvorrichtung 1 in dem Ausführungsbeispiel den zuvor genannten zusätzlichen Greiferabschnitt 37 auf. Der trichterförmige Katheter-Verbindungsadapter 5 ist mit seinem Greiferabschnitt 37 mit dem Spritzendeckel 8 verbunden. Der Greiferabschnitt 37 ist dabei beispielsweise zylindrisch ausgebildet, wie in dem Ausführungsbeispiel der Spritzenvorrichtung 1 in Fig. 3f gezeigt ist. Der Greiferabschnitt 37 weist eine Länge LG auf von vorzugsweise wenigstens 2mm, um von einer Greifereinrichtung (nicht dargestellt) erfasst zu werden, um die Spritzenvorrichtung 1 zu transportieren. Der Greiferabschnitt 37 kann aber statt einer zylindrischen Kontur auch jede andere für eine zugeordnete Greifereinrichtung geeignete Kontur aufweisen. Ein derartiger Greiferabschnitt 37 kann bei allen Ausführungsformen der erfindungsgemäßen Spritzvorrichtung 1, wie sie mit Bezug auf die Fig. 2a, 2b - 10 beschrieben werden, vorgesehen werden. Der Übergang des Greiferabschnitts 37 zu dem übrigen Spritzengehäuse 6 kann dabei zusätzlich gerundet ausgeführt werden, wie mit einer gestrichelten Linie in Fig. 3f angedeutet ist, um eine Desinfizierung der Spritzenvorrichtung 1 zusätzlich zu vereinfachen.

Grundsätzlich kann, je nach Funktion und Einsatzzweck in dem Ausführungsbeispiel in Fig. 3f auch kein Greiferabschnitt 37 vorgesehen sein. In dem Beispiel in Fig. 3f ist der trichterförmige Katheter-Verbindungsadapter 5 ab dem Greiferabschnitt 37 konisch ausgebildet. Ohne den Greiferabschnitt 37 kann der trichterförmige Verbindungsadapter 5 auch bereits ab dem Spritzendeckel 8 konisch ausgebildet sein, wie mit einer gepunkteten Linie in Fig. 3f angedeutet ist.

Die stufenlose oder mit anderen Worten glatte Außenkontur (insbesondere glatte konische oder glatte kegelstumpfförmige Außenkontur und der optionale glatte, zylindrische, oder glatte, konische äußerste End- und Befestigungsabschnitt 33, sowie der optionale glatte, vorzugweise zylindrische Greiferabschnitt 37 wie in Fig. 3f) des Katheter-Verbindungsadapters 5 hat den Vorteil, dass ein solcher trichterförmiger Katheter-Verbindungsadapter 5 sehr einfach und kostengünstig in der Herstellung ist. Des Weiteren lässt sich die stufenlose oder glatte Außenkontur des trichterförmigen Katheter-Verbindungsadapters 5, wie insbesondere in den Beispielen in den Fig. 3d, 3e und 3f gezeigt, besonders gut desinfizieren, da sich keine Verunreinigungen in Vertiefungen sammeln können. Die verschiedenen Katheter aus einem üblicherweise flexiblen oder elastischen Material, wie z. B. Gummi, Latex, PVC usw., lassen sich zudem sehr einfach auf den stufenlosen oder glatten, trichterförmigen Katheter-Verbindungsadapter 5 aufschieben, soweit bis er ausreichend fest auf dem trichterförmigen Katheter-Verbindungsadapter 5 sitzt. Übergänge zwischen dem Greiferabschnitt 37, der konischen Außenkontur und dem äußersten End- und Befestigungsabschnitt 33 des trichterförmigen Katheter-Verbindungsadapters 5, sowie ein jeweiliger Übergang zwischen dem trichterförmigen Katheter-Verbindungsadapter 5 und dem Spritzendeckel 8, können wahlweise zusätzlich gerundet ausgeführt sein, um eine Desinfizierung weiter zu erleichtern.

Zwar ist es denkbar an einem Spritzengehäuse einen Katheter-Verbindungsadapter als separates Bauteil mittels des Luer Lock-Prinzips lösbar zu befestigen. Das gesamte System muss dabei jedoch bestimmte Eigenschaften und Einschränkungen erfüllen, um die Vorabfüllung in einem pharmazeutisch technischen Standardverfahren zu ermöglichen. Solche Standardverfahren für die Vorabfüllung von Spritzenvorrichtungen bzw. deren Spritzengehäusen werden in der Regel in Nestsystemen durchgeführt, bei denen zum Befüllen Spritzenvorrichtungen verwendet werden, die in größeren Einheiten zuvor in einem Schalensystem geschachtelt und steril verpackt angeordnet sind. Folglich muss jede Spritzenvorrichtung mit ihrem Spritzengehäuse und einem daran vorgesehenen Katheter-Verbindungsadapter, die durch das Nestsystem vorgegebenen Voraussetzungen erfüllen.

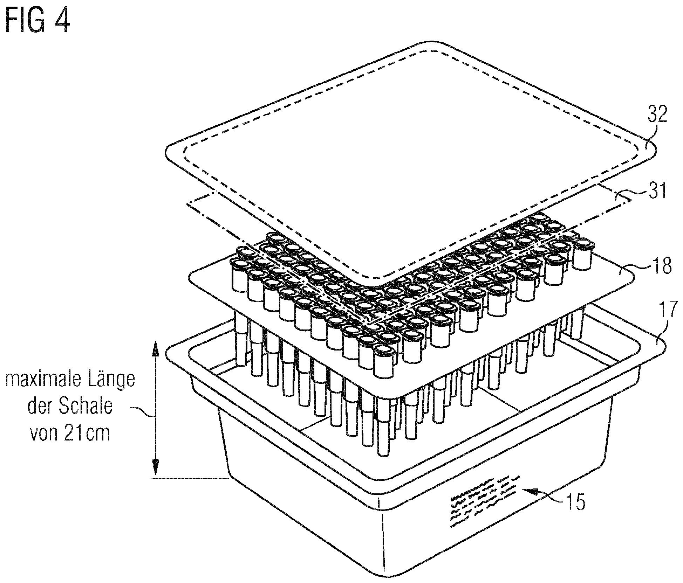

Im Nestsystem wird eine bestimmte Anzahl von leeren und sterilen Spritzenvorrichtungen, normalerweise z. B. 48 bis 100 Spritzenvorrichtungen, in einem Schalensystem 15, wie beispielhaft in Fig. 4 von der Fa. Kaatimex Ltd gezeigt, steril verpackt geliefert und direkt im Nest bzw. der Schale in einer sterilen Umgebung aufbewahrt und später befüllt.

Wie in dem Beispiel in Fig. 4 gezeigt ist, weist das jeweilige Schalensystem 15 eine Schale 17 auf, in welcher ein Träger 18 bzw. Nest eingesetzt ist, in welchem eine Einheit von Spritzenvorrichtungen aufgenommen ist. Als Spritzenvorrichtungen können dabei insbesondere die erfindungsgemäßen Spritzenvorrichtungen vorgesehen werden, wie sie mit Bezug auf die Fig. 2a, 2b und 3a-3f sowie die nachfolgenden Fig. 5-10 beschrieben sind. Der Träger 18 bzw. das Nest mit den darin aufgenommenen Spritzenvorrichtungen ist in Fig. 4 mit einem sterilen Einsatz, hier z. B. Tyvek ^{®} Einsatz 31, abgedeckt. Die Schale 17 selbst ist wiederum in Fig. 4 mit einem sterilen Deckel, hier z. B. Tyvek ^{®} Deckel 32, verschlossen.

In dem jeweiligen Befüllungsverfahren können so alle in dem Schalensystem 15 aufgenommenen Spritzenvorrichtungen, insbesondere die erfindungsgemäßen Spritzenvorrichtungen, mit dem jeweils gewünschten pharmazeutischen Produkt, z. B. einem Impfstoff, einem Krebsmedikament, einem biologischen Produkt oder, wie im vorliegenden Fall, bei einer urologischen Indikation, mit einem flüssigen Medikament, wie z. B. Oxybutinin, befüllt werden.

Eine wichtige Einschränkung bei dem Nestsystem ist dabei die Länge der herzustellenden, vorgefüllten Spritzenvorrichtung, da die Schale 17, die hierbei üblicherweise verwendet wird, eine maximale Länge von 21cm aufweist, wie in Fig. 4 angedeutet ist.

Mittels der erfindungsgemäßen Spritzenvorrichtung 1 und ihrem einstückig daran ausgebildeten, trichterförmigen Katheter-Verbindungsadapter 5, kann eine für das Nestsystem geeignete Spritzenvorrichtung 1 bereitgestellt werden, welche die maximale Länge der Schale 17 oder Nestkartusche von 21cm nicht überschreitet.

Eine solche erfindungsgemäße Spritzenvorrichtung 1, welche einstückig oder integral mit dem trichterförmigen Katheter-Verbindungsadapter 5 ausgebildet ist, kann, wie zuvor in Fig. 2a gezeigt ist, z. B., als eine 5-ml-Spritzenvorrichtung 1 mit einer Gesamtlänge von 15,5 cm (+/- 0,3 cm) mit integralem, trichterförmigem Katheter-Verbindungsadapter 5 und zusätzlichem Anschlusselement, hier Verschlusselement 14, z. B. dem Gummiverschluss, oder, wie zuvor in Fig. 2b gezeigt, z. B., als eine 10-ml-Spritze mit einer Gummikappe als Verschlusselement und einer Gesamtlänge von 20,5 cm (+/- 0,5 cm) ausgebildet werden.

Ein solcher trichterförmiger Katheter-Verbindungsadapter 5 kann einstückig oder integral an jeder Spritzenvorrichtung 1 mit einer Standard-Spritzengröße von 1 ml, 2ml, 5ml, 10ml, 20ml ausgebildet oder ausgeformt werden, da die Gesamtabmessung die Anforderungen für die nestbasierte Vorabfüllung von Spritzenvorrichtungen erfüllt. Vorgefüllte Spritzenvorrichtungen mit einer Spritzengröße von 5ml oder 10ml sind dabei die in der medizinischen Versorgung am häufigsten verwendeten Spritzenvolumen.

Der trichterförmige Katheter-Verbindungsadapter 5 kann auch einstückig mit einer Spritzenvorrichtung 1 ausgebildet sein, die eine Standardspritzengröße mit einem Volumen von mehr als 20ml, z. B. 30ml, 40ml oder 50ml usw., aufweist. Dann würde die Gesamtabmessung der Spritzenvorrichtung die zulässige Länge von 21cm für die nestbasierte Vorfüllung zwar überschreiten. In diesem Fall kann jedoch die Dimensionierung der erfindungsgemäßen Spritzenvorrichtung 1 derart geeignet angepasst werden, dass die Gesamtlänge auf z. B. maximal 20cm reduziert wird, wobei der Durchmesser des Spritzengehäuses 6 und genauer dessen Hohlzylinders 2, entsprechend vergrößert wird, um das gewünschte Volumen von mehr als 20ml, z. B. 30ml, 40ml oder 50ml usw. bereitzustellen.

Die erfindungsgemäße Spritzenvorrichtung 1 kann somit für alle gängigen Spritzengrößen durch den einstückig oder integral mit ihr ausgebildeten trichterförmigen Katheter-Verbindungsadapter 5 bereitgestellt werden und ist insbesondere auch für Spritzengrößen von mehr als 20ml, z. B. bis zu 50ml, problemlos bereitstellbar, so dass eine Handhabung und Befüllung mit dem zuvor beschriebenen gängigen Nestsystem auch bei großen Spritzenvorrichtungen weiterhin möglich ist. Insbesondere fällt bei dem einstückig oder integral mit der Spitzenvorrichtung 1 ausgebildeten trichterförmigen Katheter-Verbindungsadapter 5 die zusätzliche Verlängerung der Gesamtlänge der Spritzvorrichtung 1 durch eine Luer-Lock Verbindung weg, da der trichterförmige Katheter-Verbindungsadapter 5 unmittelbar am Ende des Spritzengehäuses 6 und dessen Spritzendeckels 8 einstückig und/oder integral mit dem Spritzendeckel 8 ausgebildet ist.

In Fig. 5 ist ein Ausschnitt einer erfindungsgemäßen Spritzenvorrichtung 1 in einer Querschnittansicht gezeigt. Die Ansicht ist dabei stark vereinfacht, rein schematisch und nicht maßstäblich dargestellt. Die Spritzenvorrichtung 1 weist einen Spritzengehäuse 6 mit einem Hohlzylinder 2 und einem Spritzendeckel 8 mit einem daran einstückig oder integral ausgebildeten trichterförmigen, abgestuften Katheter-Verbindungsadapter 5 auf. Des Weiteren weist die Spritzenvorrichtung 1 eine Kolbenanordnung 3 auf, die in Fig. 5 in einer Endposition oder komplett durchgedrückten Stellung gezeigt ist. Die Kolbenanordnung 3 in dem Ausführungsbeispiel in Fig. 5 besteht z. B. aus einem Kolben 4 und einer Kolbenstange 7.

Wie in Fig. 5 gezeigt ist, weist das Spritzengehäuse 6 der Spritzenvorrichtung 1 einen abgestuften trichterförmigen Katheter-Verbindungsadapter 5 auf, der einstückig mit dem Spritzengehäuse 6 und dessen Spritzendeckel 8 ausgebildet ist, insbesondere als Spritzgussteil. In dem Ausführungsbeispiel in Fig. 5, weist der abgestufte trichterförmige Katheter-Verbindungsadapter 5 Stufenabschnitten 13 in Form von zylindrischen Abschnitten 28 auf. Die Länge *L* bzw. Stufenhöhe der Stufenabschnitte 13, hier zylindrischen Abschnitte, können dabei identisch sein oder variieren.

Wie in Fig. 5 stark vereinfacht und rein schematisch dargestellt, wird ein Katheter 9 mit einem Befestigungs-Innendurchmesser 36, auf einen entsprechenden Stufenabschnitt 13, hier zylindrischen Abschnitt 28, des Katheter-Verbindungsadapters 5 mit einem (Außen-) Durchmesser *D*x aufgeschoben, so dass er fest auf dem Stufenabschnitt 13 sitzt, um so an dem trichterförmigen Katheter-Verbindungsadapter 5 der Spritzenvorrichtung 1 befestigt zu werden.

Für eine einfachere Desinfizierung des trichterförmigen Katheter-Verbindungsadapters 5 und seiner Stufenabschnitte 13 kann der Übergang 19 zwischen jeweils zwei Stufenabschnitten 13 wahlweise zusätzlich gerundet sein, wie exemplarisch für zwei der Stufenabschnitte 13 in dem Beispiel in Fig. 5 angedeutet ist. Dies gilt für alle Ausführungsformen der Erfindung. Ebenso kann die jeweilige Außenkante 20 der Stufenabschnitte 13 zusätzlich abgerundet sein, wie in dem nachfolgenden Ausführungsbeispiel in Fig. 6 gezeigt ist.

Die Durchgangsöffnung 10 erstreckt durch den Spritzendeckel 8 mit dem trichterförmigen Katheter-Verbindungsadapter 5 hindurch in Längsrichtung des Spritzengehäuses 6 von innen nach außen bis zu dem äußeren Ende 12 des trichterförmigen Katheter-Verbindungsadapters 5.

In dem Ausführungsbeispiel in Fig. 5 setzt sich die Durchgangsöffnung 10 beispielsweise in Längsrichtung des Spritzengehäuses 6 und damit entlang der Längsachse 11 der Spritzenvorrichtung 1 von innen nach außen aus einem trichterförmigen Abschnitt 21 und einem zylindrischen Abschnitt 22 zusammen, wobei der zylindrische Abschnitt 22 sich dabei beispielsweise über die Gesamtlänge oder im Wesentlichen die Gesamtlänge des trichterförmigen Katheter-Verbindungsadapters 5 erstreckt. Ein Totraum, in welchem ein Rest von Flüssigkeit verbleibt, wenn der Kolben 4 der Kolbenanordnung 3 der Spritzvorvorrichtung 1 komplett durchgedrückt ist, ist vorzugsweise bei der Spritzenvorrichtung 1 so klein wie möglich ausgebildet. Zumindest ein Abschnitt der Außenkontur des Spritzendeckels 8 und/oder der erste Stufenabschnitt 13 an dem inneren Ende 38 des trichterförmigen Katheter-Verbindungsadapters 5 in dem Beispiel in Fig. 5 und den nachfolgenden Beispielen in den Fig. 6-10, kann ebenfalls optional mit einem zusätzlichen Greiferabschnitt 37, wie beispielhaft in Fig. 3f gezeigt, ausgebildet werden, je nach Funktion und Einsatzzweck. Der letzte, äußerste Stufenabschnitt 13 in Fig. 5, sowie den nachfolgenden Fig. 6-10, kann zusätzlich zu der Befestigung eines zugeordneten Katheters oder alternativ dazu auch als End-und Befestigungsabschnitt ausgebildet sein zum Befestigen eines Anschlusselements, z.B. einer Injektions-Nadel oder eines Verschlusselements, wie einer Verschlusskappe. Dazu kann der End- und Befestigungsabschnitt beispielsweise zylindrisch oder als Luer-Konus ausgebildet sein, wie in dem Beispiel in Fig. 3f.

In Fig. 6 ist ein weiteres Ausführungsbeispiel einer Spritzenvorrichtung 1 gezeigt. Die Spritzenvorrichtung 1 ist dabei im Wesentlichen identisch mit der Spritzenvorrichtung 1 gemäß Fig. 5, so dass auf die Ausführungen zu Fig. 5 Bezug genommen wird, um unnötige Wiederholungen zu vermeiden. Die zylindrischen Stufenabschnitte 13 in Fig. 6 sind dabei an ihrer jeweiligen Außenkante 20 zusätzlich abgerundet. Wie beispielhaft für zwei Stufenabschnitte zuvor in Fig. 5 gezeigt wurde, können die Übergänge 19 zwischen zwei Stufenabschnitten 13 bei dem trichterförmigen Katheter-Verbindungsadapter 5 in Fig. 6 ebenfalls wahlweise zusätzlich gerundet sein (nicht dargestellt). Insbesondere können vorzugsweise alle Übergänge 19 zwischen den Stufenabschnitten 13 zusätzlich gerundet sein. Dies gilt für alle Ausführungsformen der Erfindung.

Das Ausführungsbeispiel in Fig. 6 unterscheidet sich von dem Ausführungsbeispiel in Fig. 5 dadurch, dass die Durchgangsöffnung 10 des Spritzendeckels 8 und seines trichterförmigen Katheter-Verbindungsadapters 5 als zylindrische Durchgangsöffnung 10 ausgebildet ist. Dies hat den Vorteil, dass der trichterförmige Katheter-Verbindungsadapter 5 besonders stabil ist, da die Wandstärke des Katheter-Verbindungsadapters 5 in Längsrichtung von außen, d. h. von seinem äußeren Ende 12, nach innen zunimmt. Wie in Fig. 6 angedeutet ist, ist der Kolben 4 der Kolbenanordnung 3 in einer Endposition dargestellt, in welcher die Kolbenanordnung 3 komplett durchgedrückt ist. Die Darstellung des Kolbens 4 und der Kolbenanordnung 3 in den Fig. 5 und 6 ist dabei rein schematisch und stark vereinfacht.

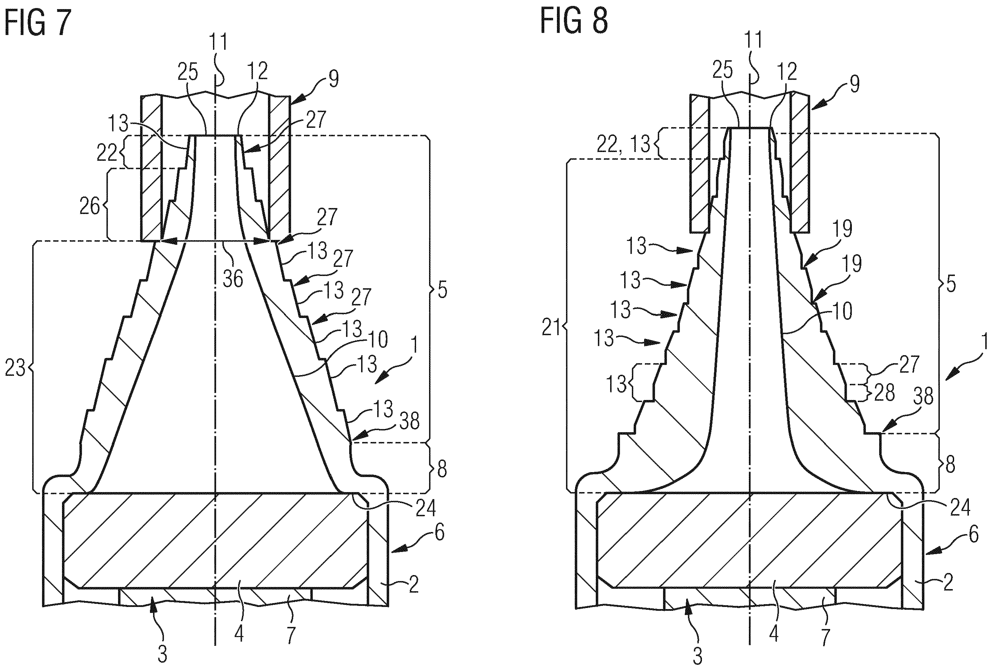

In Fig. 7 ist ein weiteres Ausführungsbeispiel einer erfindungsgemäßen Spritzenvorrichtung 1 gezeigt. Der Spritzengehäuse 6 weist dabei, wie in den vorherigen Ausführungsbeispielen in den Fig. 5 und 6, einen einstückig oder integral an ihm ausgebildeten abgestuften trichterförmigen Katheter-Verbindungsadapter 5 auf. Diesbezüglich wird auf die Ausführungen zu den Fig. 5 und 6 Bezug genommen. Die Stufenabschnitte 13 des trichterförmigen Katheter-Verbindungsadapters 5 in Fig. 7 sind aber im Gegensatz zu den Stufenabschnitten 13 in den Fig. 5 und 6 keine zylindrischen Abschnitte.

Stattdessen sind in dem Ausführungsbeispiel in Fig. 7 die Stufenabschnitte 13 jeweils als kegelstumpfförmige oder konische Abschnitte 27 ausgebildet. Die kegelstumpfförmigen oder konischen Abschnitte 27 des trichterförmigen Katheter-Verbindungsadapters 5 sind derart ausgebildet, dass sie sich in Längsrichtung und damit entlang der Längsachse 11 der Spritzenvorrichtung 1 und ihres Spritzengehäuses 6 nach außen verjüngen. Wahlweise zusätzlich können die Außenkanten 20 der Stufenabschnitte 13 zusätzlich abgerundet sein, wie z. B. in Fig. 6, und/oder die Übergange 19 zwischen den Stufenabschnitten 13 können zusätzlich gerundet sein, wie beispielhaft zuvor mit Bezug auf Fig. 5 und 6 beschrieben wurde. Dies gilt für alle Ausführungsformen der Erfindung.

In dem Ausführungsbeispiel in Fig. 7 ist ein Katheter 9 stark vereinfacht und nicht maßstäblich dargestellt, welcher mit seinem Befestigungs-Innendurchmesser 36 auf einen zugeordneten Stufenabschnitt 13, hier kegelstumpfförmigen oder konischen Abschnitt 27, des trichterförmigen Katheter-Verbindungsadapters 5 aufgeschoben und an ihm befestigt ist. Die Kegelstumpfform oder Konusform der Stufenabschnitte 13 des trichterförmigen Katheter-Verbindungsadapters 5 hat den Vorteil, dass das Aufschieben des Katheters 9 auf den trichterförmigen Katheter-Verbindungsadapter 5 zusätzlich erleichtert wird.

In dem in Fig. 7 gezeigten Ausführungsbeispiel weist die Durchgangsöffnung 10 des trichterförmigen Katheter-Verbindungsadapters 5 z. B. einen konischen oder kegelstumpfförmigen Abschnitt 23 und einen zylindrischen Abschnitt 22 auf, wobei der konische oder kegelstumpfförmige Abschnitt 23 an dem inneren Ende 24 und der zylindrische Abschnitt 22 an dem äußeren Ende 25 der Durchgangsöffnung 10 vorgesehen ist. Der konische oder kegelstumpfförmige Abschnitt 23 geht dabei in einem Übergangsbereich 26 in den zylindrischen Abschnitt 22 über. Der Übergangsbereich 26 ist optional und kann auch entfallen, je nach Funktion und Einsatzzweck.

Der Kolben 4 der Kolbenanordnung 3, wie sie in Fig. 7 gezeigt ist, ist stark vereinfacht und rein schematisch dargestellt. Er ist in Fig. 7 in seiner Endposition oder komplett durchgedrückten Position dargestellt Der Kolben 4 kann jede andere Form aufweisen und sich des Weiteren auch teilweise in die Durchgangsöffnung 10 hineinerstrecken, je nach Funktion und Einsatzzweck. Dies gilt für alle Ausführungsformen der Erfindung.

In Fig. 8 ist ein weiteres Ausführungsbeispiel einer erfindungsgemäßen Spritzenvorrichtung 1 gezeigt. Das Spritzengehäuse 6 weist dabei, wie in den Ausführungsbeispielen in Fig. 5, 6 und 7 einen einstückig oder integral an ihm ausgebildeten, abgestuften trichterförmigen Katheter-Verbindungsadapter 5 auf. Diesbezüglich wird auf die Ausführungen zu den Fig. 5, 6 und 7 Bezug genommen.

Die Stufenabschnitte 13 des trichterförmigen Katheter-Verbindungsadapters 5 in Fig. 8 sind aber im Gegensatz zu den Stufenabschnitten 13 in Fig. 7 nicht ausschließlich kegelstumpfförmig oder konisch ausgebildet, sondern der jeweilige Stufenabschnitt 13 setzt sich jeweils in Längsrichtung und damit entlang der Längsachse 11 der Spritzenvorrichtung 1 und seines trichterförmigen Katheter-Verbindungsadapters 5 von innen nach außen aus einem ersten oder (in Längsrichtung) inneren zylindrischen Abschnitt 28 und einem daran anschließenden zweiten oder (in Längsrichtung) äußeren kegelstumpfförmigen oder konischen Abschnitt 27 zusammen. Wahlweise können die Außenkanten 20 der Stufenabschnitte 13 dabei zusätzlich abgerundet sein, wie z. B. in Fig. 6, und/oder der Übergang 19 zwischen jeweils zwei Stufenabschnitten 13 zusätzlich gerundet sein, wie beispielhaft mit Bezug auf Fig. 5 und 6 beschrieben wurde. Dies gilt für alle Ausführungsformen der Erfindung.

Der in Fig. 8 dargestellte Katheter 9 wird mit seinem Befestigungs-Innendurchmesser 36 auf den jeweiligen zugeordneten Stufenabschnitt 13 aufgeschoben, wobei der kegelstumpfförmige oder konische Abschnitt 27 dabei das Aufschieben zusätzlich erleichtert, wenn der Katheter 9 auf den zylindrischen Abschnitt 28 des Stufenabschnitts 13 aufgesteckt und der Katheter 9 dadurch an dem Stufenabschnitt 13 befestigt wird.

In dem Ausführungsbeispiel in Fig. 8 setzt sich die Durchgangsöffnung 10 beispielsweise in Längsrichtung des Spritzengehäuses 6 und damit entlang der Längsachse 11 der Spritzenvorrichtung 1 von innen nach außen aus einem trichterförmigen Abschnitt 21 und einem zylindrischen Abschnitt 22 zusammen, wobei der zylindrische Abschnitt 22 sich dabei beispielsweise wenigstens oder auch nur entlang der Länge des äußersten Stufenabschnitts 13 erstreckt und der trichterförmige Abschnitt 21 sich wiederum von dem inneren Ende der Durchgangsöffnung 10 bis zu trichterförmigen Abschnitt 21 erstreckt.

Der Kolben 4 der Kolbenanordnung 3, wie sie in Fig. 8 gezeigt ist, ist ebenfalls stark vereinfacht und rein schematisch dargestellt. Dabei ist der Kolben 4 in seiner Endposition oder komplett durchgedrückt dargestellt. In Ausführungsbeispielen kann der Kolben je nach Funktion und Einsatzweck, auf beispielsweise derart ausgebildet sein, dass er in einem Teilabschnitt des trichterförmigen Abschnitts 21 aufgenommen ist, wenn er in seiner Endposition. Dies gilt für alle Ausführungsformen der Erfindung.

In den Fig. 9 und 10 ist eine ein weiteres Ausführungsbeispiel einer erfindungsgemäßen Spritzenvorrichtung 1 gezeigt. Fig. 10 zeigt dabei einen vergrößerten Ausschnitt der Spritzenvorrichtung 1 gemäß Fig. 9.

Die Kolbenanordnung ist in den Fig. 9 und 10 aus Gründen der Übersichtlichkeit weggelassen worden. Es kann aber beispielsweise eine Kolbenanordnung 3 wie zuvor z. B. in den 5 bis 8 vorgesehen werden, ohne jedoch darauf beschränkt zu sein.

Das Spritzengehäuse 6 weist, wie in den Ausführungsbeispielen zuvor in den Fig. 2a, 2b, 3a-3c, und 5 bis 8 einen einstückig an ihm ausgebildeten abgestuften trichterförmigen Katheter-Verbindungsadapter 5 auf. Diesbezüglich wird auch auf die Ausführungen zu den Fig. 2b, 3a-3f, und 5 bis 8 Bezug genommen.

Die Stufenabschnitte 13 des trichterförmigen Katheter-Verbindungsadapters 5 in den

Fig. 9 und 10 sind, wie mit einer gestrichelten Linie, aber im Gegensatz zu den Stufenabschnitten beispielsweise in Fig. 5, wie mit einer durchgezogenen Linie zum Vergleich in den Fig. 9 und 10 eingezeichnet, keine zylindrischen Abschnitte.

Stattdessen, sind die Stufenabschnitte 13 in dem Ausführungsbeispiel in den Fig. 9 und 10, wie mit der gestrichelten Linie angedeutet ist, als gerundete oder nach außen gewölbte Abschnitte 29, z. B. ballige Abschnitte, ausgebildet, auf die ein zugeordnete Katheter 9 mit seinem Befestigungs-Innendurchmesser 36, wie in Fig. 9 und 10 gezeigt, aufgeschoben werden kann. Des Weiteren ist der Übergang zwischen den Stufenabschnitten 13 und genauer den nach außen gewölbten Abschnitten 29 statt beispielsweise kantig, insbesondere scharfkantig, wahlweise zusätzlich gerundet ausgebildet. Dieser gerundete Übergang 19 zwischen den Stufenabschnitten 13 hat den Vorteil, dass er leichter sterilisiert werden kann. Die durchgängig von seinem inneren Ende 38 zu seinem äußeren Ende 12 wellenförmig ausgebildete Außenkontur des trichterförmigen Katheter-Verbindungsadapters 5 in den Fig. 9 und 10 hat den Vorteil, dass die Wellenkontur sich gut desinfizieren lässt und ein Katheter 9 mit seinem Befestigungs-Innendurchmesser 36 leicht auf die Wellenkontur des trichterförmigen Katheter-Verbindungsadapters 5 aufgeschoben werden kann, bis er fest auf diesem aufsitzt.

Die Durchgangsöffnung 10 des trichterförmigen Katheter-Verbindungsadapters 5 kann ebenfalls, wie zuvor in Fig. 8 gezeigt und beschrieben, ausgebildet sein. So kann die Durchgangsöffnung 10 beispielsweise in Längsrichtung des Spritzengehäuses 6 und damit entlang der Längsachse 11 der Spritzenvorrichtung 1 von innen nach außen aus einem trichterförmigen Abschnitt 21 und einem zylindrischen Abschnitt 22 zusammengesetzt sein, wobei der zylindrische Abschnitt 22 sich dabei beispielsweise nur entlang der Länge wenigstens des äußersten Stufenabschnitts 13 erstreckt und der trichterförmige Abschnitt sich bis zu dem zylindrischen Abschnitt erstreckt. Ebenso kann die Durchgangsöffnung 10 z. B. auch ausschließlich als zylindrische Durchgangsöffnung 10, wie zuvor in Fig. 6, oder als ausschließlich trichterförmige Durchgangsöffnung (nicht dargestellt) ausgebildet sein usw. Dies gilt für alle Ausführungsformen der Erfindung.

Die in den Fig. 2a, 2b und Fig. 5 bis 10 gezeigten Ausgestaltungen der Durchgangsöffnung 10 und die Anzahl, Form und Dimensionierung der Stufenabschnitte 13 des trichterförmigen abgestuften Katheter-Verbindungsadapters 5 in den Fig. 2a, 2b, 3a-3c und Fig. 5 bis 10 sowie die Form des stufenlosen trichterförmigen Katheter-Verbindungsadapters 5 in den Fig. 3d und 3e und 3f sind lediglich beispielhaft, insbesondere kann die Ausgestaltung der Durchgangsöffnung 10 als durchgehende zylindrische Durchgangsöffnung 10 oder als durchgehend trichterförmige Durchgangsöffnung 10 oder als eine einen zylindrischen Abschnitt 22 und einen trichterförmigen Abschnitt 21 und/oder konischen Abschnitt 23 aufweisende Durchgangsöffnung 10 ausgebildet sein usw.. Gleiches gilt für die Form, die Anzahl und Dimensionierung der Stufenabschnitte 13 des trichterförmigen abgestuften Katheter-Verbindungsadapters 5, diese können gemäß den Fig. 2a, 2b, 3a-3c und Fig. 5 bis 10 beliebig miteinander kombiniert werden zum Anschließen von verschiedenen Kathetern 9 und ihrer Kathetergröße. Des Weiteren kann der trichterförmige Katheter-Verbindungsadapter der erfindungsgemäßen Spritzenvorrichtung 1 beliebige Kombination von Stufenabschnitten 13, wie sie in den Fig. 2a, 2b, 3a-3c und Fig. 5 bis 10 beispielhaft gezeigt sind, aufweisen, sowie wahlweise zusätzlich auch wenigstens einen Bereich mit einer glatten Außenkontur, wie der Katheter-Verbindungsadapter in den Fig. 3d und 3e und 3f. Wenigstens ein, mehrere oder alle Stufenabschnitte 13 eines trichterförmigen Katheter-Verbindungsadapters 5 der erfindungsgemäßen Spritzenvorrichtung 1 kann zylindrisch (wie in Fig. 5 und 6), konisch (wie in Fig. 7) oder einen zylindrischen und einen konischen Abschnitt (wie in Fig. 8) oder zwei konische Abschnitte (wie in Fig. 3a und 3b) oder einen gerundeten oder nach außen gewölbte Abschnitt (wie in Fig. 9 und 10) oder einen Abschnitt mit einer glatten Außenkontur (wie in Fig. 3d und 3e und 3f) aufweisen. Der Übergang zwischen dem trichterförmigen Katheter-Verbindungsadapters 5 oder sofern vorhanden, Greiferabschnitt 37, und dem Spritzendeckel 8 kann ebenfalls zusätzlich gerundet ausgeführt sein, um ein Desinfizieren oder Sterilisieren zu erleichtern. Dies gilt für alle Ausführungsformen der Erfindung. Die Ausgestaltung der Durchgangsöffnung 10 und der Kolbenanordnung 3, wie zuvor in den Fig. 2a, 2b und 5-10 beispielhaft gezeigt, können bei den zuvor mit Bezug auf die Fig. 2a, 2b, 3a-3f und 5-10 gezeigten Spritzenvorrichtungen 1 mit ihrem einstückig oder integral ausgebildeten trichterförmigen Katheter-Verbindungsadapter 5 vorgesehen und beliebig kombiniert werden, insbesondere einzelne Merkmale davon, je nach Funktion und Einsatzzweck.

Die erfindungsgemäße Spritzenvorrichtung 1, wie zuvor beispielhaft in den Fig. 2a, 2b, 3a-3f und 4 bis 10 beschrieben, kann in bestimmten Ausführungsformen beispielsweise für die Katheterisierung als Selbstkatheterisierung mit urologischen Kathetern verwendet oder eingesetzt werden. Weiter kann die erfindungsgemäße Spritzenvorrichtung 1, wie zuvor beispielhaft in den Fig. 2a, 2b, 3a-3f und 4 bis 10 beschrieben, in einigen Ausführungsformen durch eine andere Person, einen unterstützenden Angehörigen oder eine Krankenschwester oder medizinisches Personal, ebenfalls unter Verwendung urologischer Katheter, für den Katheterismus verwendet oder eingesetzt werden. Anstelle von urologischen Kathetern sind zur Verwendung mit der erfindungsgemäßen Spritzenvorrichtung 1 auch andere Katheter denkbar, z. B. Drainagen aus Körperhöhlen oder beliebigen Flüssigkeits- oder Gaspools.

Die zuvor mit Bezug auf die Fig. 2a, 2b, 3a-3f und 5-10 beschriebenen Beispiele der erfindungsgemäßen Spritzenvorrichtung 1 sind miteinander beliebig kombinierbar und insbesondere einzelne Merkmale davon.

Die erfindungsgemäße Spritzenvorrichtung 1 weist vorzugsweise die in der Tabelle in Fig. 3c aufgeführten geometrischen Abmessungen für den jeweiligen (Außen-) Durchmesser D und die jeweilige Länge L bzw. Stufenhöhe der Stufenabschnitte ihres abgestuften trichterförmigen Katheter-Verbindungsadapters auf. In diesem Zusammenhang bedeutet der Begriff "tolerierbare Abweichung" eine Schwankungsbreite von ± 10 %, vorzugsweise ± 5 %, in Bezug auf die oben genannten Parameter, um eine ordnungsgemäße Anpassung der Katheter zu gewährleisten.

In bestimmten Ausführungsformen wird die pharmazeutische Zusammensetzung der vorliegenden Erfindung bei einem Katheterverfahren verwendet. In einer beispielhaften Ausführungsform entspricht die Spritzenvorrichtung der Zusammensetzung des Velariq ^{®}-Spritzensets oder ist hierfür geeignet ausgebildet.

Die erfindungsgemäße Spritzenvorrichtung, wie zuvor mit Bezug auf die Fig. 2a, 2b bis 10 beschrieben wurde, stellt ein Kombinationsprodukt bereit aus einer Spritze und einem trichterförmigen Katheter-Verbindungsadapters 5, beispielsweise einem Stufenkegeladapter wie in den Fig. 2a, 2b, 3a-3c und 5-10, oder einem glatten Kegeladapter oder einem glatten, konischen Adapter bereit, wie in den Fig. 3d, 3e und 3f beispielhaft gezeigt ist. Insbesondere kann so z.B. eine 10ml Toppac Spritzenvorrichtung aus einer Spritze, z.B. aus COC, und einem trichterförmigen Katheter-Verbindungsadapter 5, z.B. ebenfalls aus COC, wie z.B. mit Bezug auf die Fig. 2a, 2b bis 10 beschrieben, bereitgestellt werden. Das Ziel ist es, eine Erleichterung für die Patienten zu erzielen, damit diese den trichterförmigen Katheter-Verbindungsadapters, z.B. in Form eines Stufenkegeladapters oder eines glatten, konischen Adapters usw., nicht mehr eigenhändig montieren müssen. Die erfindungsgemäße Spritzenvorrichtung 1, wie zuvor beispielhaft mit Bezug auf die 2a, 2b, 3a-3c und 5-10 beschrieben, ist mit ihrem trichterförmigen Katheter-Verbindungsadapter 5 derart ausgebildet, dass an den trichterförmigen Katheter-Verbindungsadapter 5 vorzugsweise wenigstens zwei, drei, vier, fünf, sechs, sieben, acht, neun, zehn oder mehr Standardgrößen in einem Bereich von z. B. 18 bis 34 French oder entsprechend in einem Bereich von 18 bis 34 Charrière befestigbar und insbesondere flüssigkeitsdicht befestigbar sind.

Die erfindungsgemäße Spritzenvorrichtung 1 kann in ihrer Dimensionierung, insbesondere ihrer Gesamtlänge bei maximal ausgezogener Kolbenanordnung und ihrem Füllungsvolumen, problemlos angepasst werden, so dass die erfindungsgemäße Spritzenvorrichtung so reibungslos wie möglich in einen Herstellungs-Prozess integriert werden kann. Dies gilt insbesondere für die Integration der erfindungsgemäßen Spritzenvorrichtung in die Nesttechnologie und das Erfüllen der Anforderungen für die Nesttechnologie bei der automatischen Vorfüllung der Spritzenvorrichtung (engl. nested filling), bei der eine Gesamtlänge der Spritzenvorrichtung durch die Nesttechnologie vorgegeben wird.

Die erfindungsgemäße Spritzenvorrichtung kann dabei Teil eines Ready-to-Fill Systems sein, bestehend aus Doppelbeutel, Tub, Nest und 10 ml TopPac Spritze. Das sind in der urologischen Pflege übliche "Auffang-Gefäße", um Urin zu sammeln.

Die Abfüllung erfolgt auf einer Syntegon Fülllinie bestehend aus: ABO 5000, ATO 3000, FXS 2051 C. Das Tub darf eine maximale Höhe von ca. 154 mm aufweisen.

**Bezugszeichenliste**

| | | | |
|---|---|---|---|
| 1 | Spritzenvorrichtung | 36 | Befestigungs-Innendurchmesser (Katheter) |
| 2 | Hohlzylinder | 37 | Greiferabschnitt |
| 3 | Kolbenanordnung | 38 | inneres Ende (Katheter-Verbindungsadapter) |
| 4 | Kolben | | |
| 5 | Katheter-Verbindungsadapter | | |
| 6 | Spritzengehäuse | | |
| 7 | Kolbenstange | | |
| 8 | Spritzendeckel | | |
| 9 | Katheter | | |
| 10 | Durchgangsöffnung | | |
| 11 | Längsachse der Spritzenvorrichtung | | |
| 12 | äußeres Ende (Katheter-Verbindungsadapter) | | |
| 13 | Stufenabschnitt (Katheter-Verbindungsadapter) | | |
| 14 | Verschlusselement | | |
| 15 | Schalensystem | | |
| 17 | Schale | | |
| 18 | Träger | | |
| 19 | Übergang | | |
| 20 | Außenkante | | |
| 21 | trichterförmiger Abschnitt (Durchgangsöffnung) | | |
| 22 | zylindrischer Abschnitt (Durchgangsöffnung) | | |
| 23 | kegelstumpfförmiger oder konischer Abschnitt | | |
| 24 | inneres Ende (Durchgangsöffnung) | | |
| 25 | äußeres Ende (Durchgangsöffnung) | | |
| 26 | Übergangsbereich (Durchgangsöffnung) | | |
| 27 | kegelstumpfförmiger oder konischer Abschnitt | | |
| 28 | zylindrischer Abschnitt (Stufenabschnitt des Katheter-Verbindungsadapters) | | |
| 29 | nach außen gewölbter Abschnitt (Stufenabschnitt des Katheter-Verbindungsadapters) | | |
| 30 | gerundeter Übergang (Stufenabschnitt des Katheter-Verbindungsadapters) | | |
| 31 | Tyvek ^{®} Einsatz | | |
| 32 | Tyvek ^{®} Deckel | | |
| 33 | äußerer Endabschnitt | | |
| 34 | erster konischer Abschnitt | | |
| 35 | zweiter konischer Abschnitt | | |

## Patentansprüche

1. Spritzenvorrichtung (1), die zur Injektion einer Flüssigkeit, insbesondere eines flüssigen Medikaments, oder zum Abnehmen einer Flüssigkeit dient, wobei die Spritzenvorrichtung (1) aufweist:
ein Spritzengehäuse (6) mit einem Spritzendeckel (8), wobei der Spritzendeckel (8) einstückig mit einem trichterförmigen Katheter-Verbindungsadapter (5) ausgebildet ist, wobei der trichterförmige Katheter-Verbindungsadapter (5) sich in Längsrichtung der Spritzenvorrichtung (1) von einem inneren, dem Spritzengehäuse (6) zugewandten Ende zu einem äußeren, dem Spritzengehäuse (6) abgewandten Ende (12) hin verjüngt,
wobei die Außenkontur des trichterförmigen Katheter-Verbindungsadapters (5) in Längsrichtung abgestuft mit mehreren Stufenabschnitten (13) ausgebildet ist zum Befestigen von Kathetern mit unterschiedlichen Befestigungs-Innendurchmessern,
wobei der Übergang (19) zwischen wenigstens zwei der mehreren oder zwischen allen Stufenabschnitten (13) gerundet ist, **dadurch gekennzeichnet, dass** zusätzlich wenigstens zwei der mehreren Stufenabschnitte (13) des trichterförmigen Katheter-Verbindungsadapters (5) nach außen gewölbt ausgebildet sind, zur Ausbildung einer wellenförmigen
oder rippenförmigen Außenkontur des trichterförmigen Katheter-Verbindungsadapters (5).

2. Spritzenvorrichtung nach Anspruch 1, wobei wenigstens ein oder mehrere Stufenabschnitte (13) des trichterförmigen Katheter-Verbindungsadapters (5) jeweils zylindrisch (28) ausbildet ist bzw. sind.

3. Spritzenvorrichtung nach Anspruch 1 oder 2, wobei wenigstens ein oder mehrere Stufenabschnitte (13) des trichterförmigen Katheter-Verbindungsadapters (5) konisch (27) ausgebildet ist bzw. sind und sich in Längsrichtung der Spritzenvorrichtung (1) von innen nach außen konisch verjüngt bzw. verjüngen.

4. Spritzenvorrichtung nach einem der Ansprüche 1 bis 3, wobei wenigstens ein oder mehrere Stufenabschnitte (13) des trichterförmigen Katheter-Verbindungsadapters (5) einen ersten oder in Längsrichtung der Spritzenvorrichtung (1) inneren, zylindrisch ausgebildeten Abschnitt (28) und einen daran anschließenden zweiten oder äußeren, konisch ausgebildeten Abschnitt (27) aufweist bzw. aufweisen, wobei der zweite oder äußere, konisch ausgebildete Abschnitt (27) sich in Längsrichtung der Spritzenvorrichtung (1) von innen nach außen konisch verjüngt.

5. Spritzenvorrichtung nach einem der Ansprüche 1 bis 4, wobei wenigstens ein oder mehrere Stufenabschnitte (13) des trichterförmigen Katheter-Verbindungsadapters (5) einen ersten oder in Längsrichtung der Spritzenvorrichtung (1) inneren, konisch ausgebildeten Abschnitt (34) und einen daran anschließenden zweiten oder äußeren, konisch ausgebildeten Abschnitt (35) aufweist bzw. aufweisen, wobei der erste und zweite konisch ausgebildete Abschnitt (34, 35) sich jeweils in Längsrichtung der Spritzenvorrichtung (1) von innen nach außen konisch verjüngen, wobei der zweite oder äußere, konisch ausgebildete Abschnitt (35) sich dabei stärker in Längsrichtung der Spritzenvorrichtung (1) von innen nach außen konisch verjüngt als der erste, konisch ausgebildete Abschnitt (34).

6. Spritzenvorrichtung nach einem der Ansprüche 2 bis 5, wobei eine Außenkante (20) wenigstens eines Abschnitts des wenigstens einen oder der mehreren Stufenabschnitte (13) zusätzlich abgerundet ist oder als abgerundete Außenkante ausgebildet ist.

7. Spritzenvorrichtung nach Anspruch 1, wobei alle Stufenabschnitte (13) des trichterförmigen Katheter-Verbindungsadapters (5) nach außen gewölbt ausgebildet ist bzw. sind.

8. Spritzenvorrichtung nach Anspruch 1, wobei alle Stufenabschnitte (13) des trichterförmigen Katheter-Verbindungsadapters (5) nach außen gewölbt ausgebildet sind und der Übergang (19) zwischen allen Stufenabschnitten (13) zusätzlich gerundet ist zur Ausbildung einer wellenförmigen oder rippenförmigen Außenkontur des trichterförmigen Katheter-Verbindungsadapters (5).

9. Spritzenvorrichtung nach Anspruch 1 oder 8, wobei die wenigstens zwei oder alle Stufenabschnitte (13) des trichterförmigen Katheter-Verbindungsadapters (5) ballig, halbrund oder als abgerundete Rippe nach außen gewölbt ausgebildet ist bzw. sind.

10. Spritzenvorrichtung nach einem der Ansprüche 1 bis 6,
wobei ein äußerster Stufenabschnitt (13) des trichterförmigen Katheter-Verbindungsadapters (5) zum Befestigen eines Katheters (9) zusätzlich zum Befestigen eines Anschlusselements, ausgebildet ist und besonders bevorzugt zylindrisch und/oder konisch, insbesondere als Luer-Konus, ausgebildet ist, wobei das Anschlusselement vorzugsweise eine Injektions-Nadel oder ein Verschlusselement, insbesondere eine Verschlusskappe (14) ist.

11. Spritzenvorrichtung nach einem der Ansprüche 1 bis 10,
wobei der trichterförmige Katheter-Verbindungsadapters (5) und/oder der Spritzendeckel (8) einen Greiferabschnitt (37) aufweisen zum Greifen der Spritzenvorrichtung, und/oder
wobei das Spritzengehäuse (6) eine Durchgangsöffnung (10) aufweist, die sich durch den Spritzendeckel (6) mit dem trichterförmigen Katheter-Verbindungsadapter (5) bis zu dem äußeren Ende (12) hindurch erstreckt, wobei die Durchgangsöffnung (10) insbesondere zylindrisch (22) und/oder trichterförmig (21) ausgebildet ist.

12. Spritzenvorrichtung nach einem der Ansprüche 1 bis 11, wobei die Spritzenvorrichtung (1) eine Gesamtlänge von weniger als 21cm und insbesondere höchstens 20,5cm aufweist, wenn die Kolbenanordnung (3) in eine Endposition ausgezogen ist, in welcher die Spritzenvorrichtung (1) ein maximales, vorbestimmtes Spritzenvolumen aufweist, beispielsweise ein Spritzenvolumen in einem Bereich von vorzugsweise 1ml bis zu 20ml und besonders bevorzugt von 1ml bis 50ml, und/oder wobei das Spritzengehäuse (6) mit dem Spritzendeckel (8) und dem trichterförmigen Katheter-Verbindungsadapter (5) einstückig aus Kunststoff, Metall und/oder Glas ausgebildet ist und insbesondere als Kunststoffspritzgussteil, wobei das Kunststoffspritzgussteil insbesondere ein Einkomponenten-Spritzgussteil oder wenigstens ein Zwei-Komponenten-Spritzgussteil oder ein Dreikomponenten-Spritzgussteil ist.

13. Spritzenvorrichtung, insbesondere 1ml, 5ml, 10ml, 20ml, 30ml, 40ml oder 50ml Spritzenvorrichtung, nach einem der Ansprüche 1 bis 12, wobei die Spritzenvorrichtung (1) mit einem flüssigen Medikament, insbesondere mit Oxybutyninhydrochlorid für vorgefüllt und mit einem Verschlusselement verschlossen ist und/oder wobei die Gesamtlänge der vorgefüllten Spritzenvorrichtung (1) mit ihrer Kolbenanordnung (3) und ihrem Anschlusselement, insbesondere Verschlusselement, beispielsweise Verschlusskappe (14), weniger als 21cm und insbesondere höchstens 20,5cm beträgt.

## Claims

1. A syringe device (1) for injecting a liquid, in particular a liquid medicament, or for drawing off a liquid, wherein the syringe device (1) comprises:
a syringe housing (6) with a syringe cap (8), wherein the syringe cap (8) is formed integrally with a funnel-shaped catheter connection adapter (5), wherein the funnel-shaped catheter connection adapter (5) tapers in the longitudinal direction of the syringe device (1) from an inner end facing the syringe housing (6) towards an outer end (12) facing away from the syringe housing (6),
wherein the outer contour of the funnel-shaped catheter connection adapter (5) is formed with multiple stepped sections (13) in the longitudinal direction for securing catheters with different inner securing diameters, wherein the transition (19) between at least two of the stepped sections (13) or between all of them is rounded,
**characterized in that**, in addition, at least two of the plurality of stepped sections (13) of the funnel-shaped catheter connection adapter (5) are formed to curve outwards, to form a wave-shaped or ribbed outer contour of the funnel-shaped catheter connection adapter (5).

2. Syringe device according to claim 1, wherein at least one or more stepped sections (13) of the funnel-shaped catheter connection adapter (5) are each formed cylindrically (28).

3. A syringe assembly according to claim 1 or 2, wherein at least one or more stepped sections (13) of the funnel-shaped catheter connection adapter (5) are conical (27) and taper conically from the inside to the outside in the longitudinal direction of the syringe assembly (1).

4. A syringe assembly according to any one of claims 1 to 3, wherein at least one or more stepped sections (13) of the funnel-shaped catheter connection adapter (5) comprise a first or, in the longitudinal direction of the syringe assembly (1) and a second or outer, conically shaped section (27) adjoining it, wherein the second or outer, conically shaped section (27) tapers conically from the inside to the outside in the longitudinal direction of the syringe device (1).

5. A syringe assembly according to any one of claims 1 to 4, wherein at least one or more stepped sections (13) of the funnel-shaped catheter connection adapter (5) comprise a first, or inner, conically shaped section (34) in the longitudinal direction of the syringe assembly (1) and a second or outer conical section (35) adjoining it, wherein the first and second conical sections (34, 35) each taper conically from the inside to the outside in the longitudinal direction of the syringe device (1), the second or outer conically shaped section (35) tapering more sharply from the inside to the outside in the longitudinal direction of the syringe device (1) than the first conically shaped section (34).

6. Syringe device according to any one of claims 2 to 5, wherein an outer edge (20) of at least one section of the at least one or more stepped sections (13) is additionally rounded or is formed as a rounded outer edge.

7. An injection device according to claim 1, wherein all stepped sections (13) of the funnel-shaped catheter connection adapter (5) are formed to be outwardly curved.

8. An injection device according to claim 1, wherein all the stepped sections (13) of the funnel-shaped catheter connection adapter (5) are formed to be outwardly curved and the transition (19) between all stepped sections (13) is additionally rounded to form a wave-shaped or ribbed outer contour of the funnel-shaped catheter connection adapter (5).

9. A syringe assembly according to claim 1 or 8, wherein the at least two or all stepped sections (13) of the funnel-shaped catheter connection adapter (5) are formed to be convex, semicircular or rounded ribs curving outwards.

10. A syringe assembly according to any one of claims 1 to 6,
wherein an outermost stepped section (13) of the funnel-shaped catheter connection adapter (5) is designed to secure a catheter (9) in addition to securing a connecting element, and is, in a particularly preferred form, cylindrical and/or conical, in particular as a Luer cone, wherein the connecting element is preferably an injection needle or a closure element, in particular a closure cap (14).

11. A syringe assembly according to any one of claims 1 to 10,
wherein the funnel-shaped catheter connection adapter (5) and/or the syringe cap (8) comprise a gripping section (37) for gripping the syringe assembly, and/or wherein the syringe housing (6) comprises a through-opening (10) which extends through the syringe cover (6) from the funnel-shaped catheter connection adapter (5) to the outer end (12), the through-opening (10) being, in particular, cylindrical (22) and/or funnel-shaped (21).

12. Syringe device according to any one of claims 1 to 11, wherein the syringe device (1) has a total length of less than 21 cm and, in particular, not more than 20.5 cm when the plunger assembly (3) is extended to an end position in which the syringe device (1) has a maximum, predetermined syringe volume, for example a syringe volume in a range of preferably 1 ml to 20 ml and particularly preferably from 1 ml to 50 ml, and/or wherein the syringe housing (6), together with the syringe cap (8) and the funnel-shaped catheter connection adapter (5), is formed integrally from plastic, metal and/or glass, and in particular as a plastic injection-moulded part, wherein the plastic injection-moulded part is, in particular, a single-component injection-moulded part or at least a two-component injection-moulded part or a three-component injection-moulded part.

13. Syringe assembly, in particular a 1 ml, 5 ml, 10 ml, 20 ml, 30 ml, 40 ml or 50 ml syringe assembly, according to any one of claims 1 to 12, wherein the syringe assembly (1) is pre-filled with a liquid medicament, in particular with oxybutynin hydrochloride, and is sealed with a closure element and/or
whereby the total length of the pre-filled syringe assembly (1), including its piston assembly (3) and its connecting element - in particular a closure element, such as a cap (14) - is less than 21 cm and, in particular, no more than 20.5 cm.

## Revendications

1. Dispositif de seringue (1) destiné à l'injection d'un liquide, en particulier d'un médicament liquide, ou au prélèvement d'un liquide, le dispositif de seringue (1) comprenant :
un corps de seringue (6) muni d'un capuchon (8), ledit capuchon (8) étant formé d'un seul tenant avec un adaptateur de raccordement de cathéter (5) en forme d'entonnoir,
ledit adaptateur de raccordement de cathéter en forme d'entonnoir (5) se rétrécissant dans le sens longitudinal du dispositif de seringue (1), depuis une extrémité intérieure tournée vers le corps de seringue (6) jusqu'à une extrémité extérieure (12) opposée au corps de seringue (6),
le contour extérieur de l'adaptateur de raccordement de cathéter en forme d'entonnoir (5) étant réalisé avec plusieurs sections étagées (13) dans le sens longitudinal pour la fixation de cathéters présentant différents diamètres intérieurs de fixation, la transition (19) entre au moins deux de ces sections étagées ou entre toutes les sections étagées (13) étant arrondie, **caractérisé en ce qu'**en outre, au moins deux des multiples sections étagées (13) de l'adaptateur de raccordement de cathéter en forme d'entonnoir (5) sont bombées vers l'extérieur, afin de former un contour extérieur ondulé ou nervuré de l'adaptateur de raccordement de cathéter en forme d'entonnoir (5).

2. Dispositif de seringue selon la revendication 1, dans lequel au moins une ou plusieurs sections en gradins (13) de l'adaptateur de raccordement de cathéter en forme d'entonnoir (5) sont chacune de forme cylindrique (28).

3. Dispositif de seringue selon la revendication 1 ou 2, dans lequel au moins une ou plusieurs sections en gradins (13) de l'adaptateur de raccordement de cathéter en forme d'entonnoir (5) sont de forme conique (27) et se rétrécissent de manière conique de l'intérieur vers l'extérieur dans le sens longitudinal du dispositif de seringue (1).

4. Dispositif de seringue selon l'une des revendications 1 à 3, dans lequel au moins une ou plusieurs sections étagées (13) de l'adaptateur de raccordement de cathéter en forme d'entonnoir (5) comportent une première section, ou une section (28) de forme cylindrique située dans le sens longitudinal du dispositif de seringue (1) et une deuxième partie, ou partie extérieure, de forme conique (27) qui s'y raccorde, la deuxième partie, ou partie extérieure, de forme conique (27) se rétrécissant de manière conique de l'intérieur vers l'extérieur dans le sens longitudinal du dispositif de injection (1).

5. Dispositif de injection selon l'une des revendications 1 à 4, dans lequel au moins une ou plusieurs sections étagées (13) de l'adaptateur de raccordement de cathéter en forme d'entonnoir (5) comportent une première section, ou une section de forme conique située à l'intérieur dans le sens longitudinal du dispositif de injection (1) et une deuxième partie conique, ou partie extérieure (35), qui s'y raccorde, la première et la deuxième parties coniques (34, 35) se rétrécissant chacune de manière conique de l'intérieur vers l'extérieur dans le sens longitudinal du dispositif de seringue (1), la deuxième partie conique (35), ou partie extérieure, se rétrécissant alors plus fortement de l'intérieur vers l'extérieur dans le sens longitudinal du dispositif de seringue (1) que la première partie conique (34).

6. Dispositif d'injection selon l'une des revendications 2 à 5, dans lequel un bord extérieur (20) d'au moins une partie de la ou des sections étagées (13) est en outre arrondi ou est réalisé sous la forme d'un bord extérieur arrondi.

7. Dispositif d'injection selon la revendication 1, dans lequel toutes les sections en gradins (13) de l'adaptateur de raccordement de cathéter en forme d'entonnoir (5) sont bombées vers l'extérieur.

8. Dispositif d'injection selon la revendication 1, dans lequel toutes les sections en gradins (13) de l'adaptateur de raccordement de cathéter en forme d'entonnoir (5) sont bombées vers l'extérieur et la transition (19) entre toutes les sections étagées (13) est en outre arrondie afin de former un contour extérieur ondulé ou nervuré de l'adaptateur de raccordement de cathéter en forme d'entonnoir (5).

9. Dispositif de seringue selon la revendication 1 ou 8, dans lequel les au moins deux ou toutes les sections étagées (13) de l'adaptateur de raccordement de cathéter en forme d'entonnoir (5) sont bombées, semi-circulaires ou formées en nervure arrondie bombée vers l'extérieur.

10. Dispositif d'injection selon l'une des revendications 1 à 6,
dans lequel une section étagée la plus externe (13) de l'adaptateur de raccordement de cathéter en forme d'entonnoir (5) est conçu pour la fixation d'un cathéter (9) en plus de la fixation d'un élément de raccordement, et est de préférence de forme cylindrique et/ou conique, en particulier sous la forme d'un cône de Luer, l'élément de raccordement étant de préférence une aiguille d'injection ou un élément de fermeture, en particulier un capuchon de fermeture (14).

11. Dispositif de seringue selon l'une des revendications 1 à 10,
dans lequel l'adaptateur de raccordement de cathéter en forme d'entonnoir (5) et/ou le capuchon de seringue (8) comportent une partie de préhension (37) destinée à saisir le dispositif de seringue, et/ou
dans lequel le corps de seringue (6) comporte une ouverture traversante (10) qui s'étend à travers le capuchon de seringue (6) depuis l'adaptateur de raccordement de cathéter en forme d'entonnoir (5) jusqu'à l'extrémité extérieure (12), l'ouverture traversante (10) étant notamment de forme cylindrique (22) et/ou en forme d'entonnoir (21).

12. Dispositif de seringue selon l'une des revendications 1 à 11, le dispositif de seringue (1) présentant une longueur totale inférieure à 21 cm et, en particulier, d'au plus 20,5 cm lorsque l'ensemble piston (3) est tiré jusqu'à une position finale dans laquelle le dispositif de seringue (1) présente un volume d'injection maximal prédéterminé , par exemple un volume d'injection compris de préférence entre 1 ml et 20 ml et de manière particulièrement préférée entre 1 ml et 50 ml, et/ou
le boîtier de seringue (6) étant formé d'un seul tenant avec le capuchon de seringue (8) et l'adaptateur de raccordement de cathéter en forme d'entonnoir (5) en matière plastique, de métal et/ou de verre, et notamment sous la forme d'une pièce moulée par injection en plastique, ladite pièce moulée par injection en plastique étant notamment une pièce moulée par injection à un composant, ou au moins une pièce moulée par injection à deux composants, ou une pièce moulée par injection à trois composants.

13. Dispositif de seringue, en particulier dispositif de seringue de 1 ml, 5 ml, 10 ml, 20 ml, 30 ml, 40 ml ou 50 ml, selon l'une des revendications 1 à 12, le dispositif de seringue (1) étant prérempli d'un médicament liquide, en particulier de chlorhydrate d'oxybutynine, et fermé par un élément de fermeture et/ou
la longueur totale du dispositif de seringue préremplie (1), avec son ensemble piston (3) et son élément de raccordement, en particulier son élément de fermeture, par exemple un capuchon de fermeture (14), étant inférieure à 21 cm et, en particulier, ne dépassant pas 20,5 cm.
